# EUROPÄISCHE PATENTSCHRIFT

(11) **EP 3 949 080 B1**
(45) Veröffentlichungstag und Bekanntmachung des Hinweises auf die Patenterteilung: **30.11.2022**
(21) Anmeldenummer: 20717820.3
(22) Anmeldetag: 02.04.2020
(51) Int. Cl.: H02J 50/30, A61N 1/378, H02J 50/00

(54) **VORRICHTUNG ZUR ENERGIEVERSORGUNG EINES AKTIVEN AUGENIMPLANTATS**
APPARATUS FOR SUPPLYING ENERGY TO AN ACTIVE EYE IMPLANT
DISPOSITIF D'ALIMENTATION EN ÉNERGIE D'UN IMPLANT OCULAIRE ACTIF

(30) Priorität: 03.04.2019 DE 102019108678
(43) Veröffentlichungstag der Anmeldung: 09.02.2022
(73) Patentinhaber: Carl Zeiss AG, 73447 Oberkochen (DE); Carl Zeiss Jena GmbH, 07745 Jena (DE)
(72) Erfinder: NOBIS, Thomas, 04315 Leipzig (DE); SCHMITT-MANDERBACH, Tobias, 87439 Kempten (DE); HILLENBRAND, Matthias, 07745 Jena (DE); VOJTISEK, Petr, 07745 Jena (DE)
(74) Vertreter: Neusser, Sebastian
(86) Internationale Anmeldenummer: PCT/EP2020/059409
(87) Internationale Veröffentlichungsnummer: WO 2020/201427

(56) Entgegenhaltungen:
- DE-A1-102016 103 285
- DE-A1-102016 115 938
- DE-A1-102017 107 346
- US-A1- 2015 182 748

## Beschreibung

Die vorliegende Anmeldung betrifft Vorrichtungen und Verfahren zum Versorgen eines aktiven Augenimplantats mit Energie mittels Licht.

Aktive Augenimplantate sind Vorrichtungen, welche in ein Auge eines Patienten implantiert werden, um dort bestimmte Funktionen auszuüben. Ein Beispiel für derartige aktive Augenimplantate sind Retinaimplantate. Retinaimplantate werden entwickelt, um Personen, denen die Sehfähigkeit abhandengekommen ist, bei welchen jedoch noch eine Verbindung von dem Sehnerv zum Gehirn besteht, das Sehvermögen zumindest bis zu einem gewissen Grad wiederzugeben. Derartige Retinaimplantate umfassen üblicherweise einen Bildsensor, durch welchen - gegebenenfalls mit zusätzlichen Schaltungen - elektrische Impulse erzeugt werden, die dann über den Sehnerv registriert werden.

Andere Beispiele für aktive Augenimplantate sind aktiv akkommodierende Intraokularlinsen oder implantierte Sensoren zur Messung von Parametern im Auge, beispielsweise des Blutzuckerspiegels im Kammerwasser. Derartige aktive Augenimplantate benötigen im Gegensatz zu passiven Implantaten (z. B. einfachen Linsen) elektrische Energie, um betrieben zu werden.

Eine Möglichkeit der Versorgung mit Energie ist die Zuführung von Licht, beispielsweise von Infrarotstrahlung unterhalb des sichtbaren Bereichs, welche dann durch das aktive Augenimplantat im Wesentlichen mittels einer Solarzelle oder einer ähnlichen Einrichtung in elektrische Energie umgewandelt wird. Aber auch eine Versorgung mit anderen Lichtwellenlängen ist grundsätzlich möglich.

Auch kann es möglich sein, dass die durch die Strahlung übertragene Energie der Kommunikation mit dem aktiven Augenimplantat dient, beispielsweise indem die Intensität und/oder Frequenz der übertragenen Strahlung moduliert wird.

Einige Vorrichtungen und Verfahren zum Versorgen eines aktiven Augenimplantats sind in den Druckschriften US 2015 / 0 182 748 A1, DE 10 2016 103 285 A1 und DE 10 2017 107 346 A1 beschrieben.

Eine Schnittstelle zwischen externen optischen Systemen zur Versorgung eines aktiven Augenimplantats und dem menschlichen Auge mit dem betreffenden Implantat muss eine Reihe von Anforderungen erfüllen. Diese basieren zum Beispiel auf anatomischen Merkmalen des menschlichen Auges, auf den üblichen Sehgewohnheiten im betrachteten Anwendungsfall oder auf Anforderungen zur gesundheitlichen Unbedenklichkeit der eingesetzten Strahlung.

Folgende Anforderungen für ein Energieversorgungsystem für ein aktives Augenimplantat kommen, je nach Anwendungsfall, einzeln oder in Kombination in Frage.

Es ist erstrebenswert, wenn ein Energieversorgungssystem, welches beispielsweise in ein Brillengestell eingebaut wird, zuverlässig auch bei Positionierungsungenauigkeiten der Brillenfassung arbeitet. Hierfür ist beispielsweise eine Robustheit gegenüber lateralem Versatz des Auges zum optischen System und/oder eine Robustheit gegenüber axialem Versatz des Auges zum optischen System wünschenswert.

Ferner ist es erstrebenswert, dass das Energieversorgungssystem unempfindlich gegenüber physiologischen Vorgängen ist, beispielsweise eine Robustheit gegenüber Variationen der Größe der Augenpupille und/oder eine Robustheit gegenüber Variationen des Drehwinkels des Auges in der Augenhöhle aufweist. In anderen Worten sollte eine Energieversorgung des Augenimplantats bei verschiedenen Drehwinkeln oder Pupillengrößen sichergestellt sein.

Ferner ist eine hohe Effizienz des zur Verfügung gestellten Lichts erstrebenswert, d.h. möglichst eine ausschließliche Beleuchtung des oder der Lichtempfänger mit wenig Überstrahlung, um eine gute Versorgung sicherzustellen und den Energiebedarf des Systems, beispielsweise im Hinblick auf portable Systeme, zu verringern.

Es ist daher eine Aufgabe, verbesserte Vorrichtungen und Verfahren zur Versorgung eines aktiven Augenimplantats über einen großen Winkelbereich bereitzustellen.

Diese Aufgabe wird von den Vorrichtungen gemäß Anspruch 1 und den Verfahren gemäß Anspruch 14 gelöst. Die abhängigen Ansprüche definieren bevorzugte Ausführungsbeispiele.

Gemäß einem ersten Aspekt wird eine Vorrichtung mit einem Brillenglas zur Versorgung eines aktiven Augenimplantats in einem Auge eines Benutzers mit Energie bereitgestellt. Die Vorrichtung umfasst:
eine Lichtquelle,
einen Strahlexpander, der in oder auf dem Brillenglas angeordnet ist, und ein erstes Expansionselement und ein zweites Expansionselement umfasst.

Hierbei ist die Vorrichtung eingerichtet, Licht von der Lichtquelle in das Brillenglas einzukoppeln und zu dem ersten Expansionselement zu leiten.

Das erste Expansionselement ist ferner eingerichtet, das Licht zu empfangen, entlang einer ersten Richtung aufzuweiten und zumindest einen Teil des Lichts als expandiertes Licht in einer zweiten Richtung an das zweite Expansionselement zu leiten. Hierbei ist die zweite Richtung von der ersten Richtung verschieden.

Das zweite Expansionselement ist eingerichtet, das expandierte Licht zu empfangen, entlang der zweiten Richtung aufzuweiten und über eine Abgabefläche als zweifach expandiertes Licht in eine dritte Richtung bereitzustellen, wobei die dritte Richtung zumindest teilweise nicht in dem Brillenglas verläuft.

Das zweite Expansionselement ist ferner eingerichtet, das zweifach expandierte Licht effektiv zu fokussieren.

Verschiedene zuvor und nachfolgend erwähnte optische Elemente, beispielsweise das erste und zweite Expansionselement, aber auch nachfolgend erwähnte optische Elemente können als lineare Gitter mit veränderlichen Abmessungen ausgeführt werden. Sie können auch als Volumenhologramme, beispielsweise als mehrfachbelichtete Volumenhologramme und/oder als Gitter, beispielsweise als Oberflächengitter, beispielsweise als segmentierte Oberflächengitter, ausgeführt sein.

Es gibt zahlreiche weitere Möglichkeiten solche optischen Elemente zu realisieren. Beispielsweise können auch Spiegel verwendet werden. Solche Spiegel können beispielsweise in einer Anordnung, beispielsweise als ein regelmäßiges oder unregelmäßiges Array, in das Brillenglas eingebettet werden.

Bei einigen optischen Elementen kann eine fokussierende Wirkung direkt durch das Element selbst erzeugt werden, beispielsweise bei Volumenhologrammen oder bei gewölbten Spiegeln oder verzerrten Gittern. Bei anderen optischen Elementen ist eine Kombination mit weiteren optischen Elementen möglich, um eine effektive Fokussierung zu erzielen, beispielsweise mit Linsen.

In manchen Ausführungsformen der optischen Elemente können sich die jeweiligen optischen Elemente teilweise oder ganz überlappen. Beispielsweise können erstes und zweites Expansionselement teilweise oder ganz in demselben Volumen des Brillenglases angeordnet sein. Auch ein projizierter teilweiser oder vollständiger Überlapp ist möglich, beispielsweise indem ein Gitter auf einer Vorderseite eines Wellenleiters und ein zweites Gitter auf einer Rückseite eines Wellenleiters angeordnet ist, wobei der Wellenleiter ein Teil des Brillenglases sein kann und sich erstes und zweites Gitter aus einer bestimmten Richtung aus, beispielsweise beim geraden Blick durch das Brillenglas gesehen, zu überlappen scheinen, obwohl sie in verschiedenen Volumina des Brillenglases angeordnet sind.

Optische Elemente können teilweise oder vollständig im Brillenglas vergraben sein, das heißt vollständig oder teilweise von Material des Brillenglases umgeben sein.

Strahlexpander, die auf Basis von Volumenhologrammen arbeiten, sind z. B. aus der DE 10 2016 115 938 A1 oder der US 2016/0231568 A1 für sich genommen für einen anderen Zweck, nämlich in Vorrichtungen zur Dateneinspiegelung, bekannt und werden als Pupillenexpander bezeichnet. Solche bekannten Strahlexpander zum Zweck der Dateneinspiegelung bieten jedoch keine effektive Fokussierung, sondern kollimiertes oder divergierendes Licht, um ein scharfes Abbilden von Pixeln, die die eingespiegelten Daten repräsentieren, zu ermöglichen.

Für eine Energieversorgung von aktiven Augenimplantaten ist es hingegen nicht immer erforderlich, eine scharfe Abbildung einzelner Pixel zu gewährleisten. Somit können die bekannten Strahlexpander so modifiziert werden, dass sie eine effektive Fokussierung wie nachfolgend und zuvor beschrieben herbeiführen.

Solche Strahlexpander können den Vorteil haben, dass durch die Strahlexpander ein großer Winkelbereich ausgeleuchtet werden kann, so dass die Energieversorgung des aktiven Augenimplantats robust gewährleistet wird, beispielsweise bei den oben beschriebenen physiologischen Vorgängen wie Drehung des Auges und/oder Positionierungenauigkeiten der Vorrichtung. Durch die effektive Fokussierung können die Strahlexpander ferner den Vorteil haben, dass die Energieversorgung des aktiven Augenimplantats weiter verbessert wird, beispielsweise bei Pupillengrößenänderungen.

Unter Richtungen werden hierbei effektive Richtungen verstanden, die nicht eine Ausbreitungsrichtung einzelner Photonen oder Wellen charakterisieren müssen, sondern eine allgemeine, ggf. gemittelte Ausbreitungsrichtung des jeweiligen Lichtes charakterisieren können. Erfolgt beispielsweise die Ausbreitung in die zweite Richtung in dem Brillenglas mittels Totalreflexion, so beschreibt die zweite Richtung die Ausbreitungsrichtung im Brillenglas, nicht den Lichtweg einzelner, reflektierter Strahlen. Gleiches gilt für die anderen zuvor und nachfolgend genannten Richtungen entsprechend.

Unter effektivem Fokussieren wird im Rahmen dieser Anmeldung verstanden, dass das zweifach expandierte Licht auf eine gedachte Fokusfläche zwischen Abgabefläche und dem aktiven Augenimplantat entlang der dritten Richtung gelenkt wird, wobei die gedachte Fokusfläche kleiner als die Abgabefläche ist.

Die Lichtquelle kann eine Infrarotlichtquelle sein. Eine Infrarotlichtquelle kann bevorzugt im Wesentlichen (d. h. z. B. bis auf unerwünschte Effekte, z. B. unvollständige Filterung) nur im infraroten Bereich mit Wellenlängen von über 780 nm emittieren, um die Wahrnehmung von sichtbarem Licht nicht zu stören.

Die Vorrichtung kann eine kopfgetragene Vorrichtung sein, die Vorrichtung kann beispielsweise eine Brille sein. Die Vorrichtung kann auch mehrere Komponenten umfassen, beispielsweise kann die Vorrichtung eine Brille und eine Versorgungsvorrichtung umfassen die miteinander gekoppelt sind. Beispielsweise kann die Lichtquelle in der Versorgungsvorrichtung angeordnet sein und das Licht mittels eines Lichtleiters an die Brille weitergeleitet werden. Aber auch andere Ausführungen der Vorrichtung sind möglich.

Das erste Expansionselement und/oder das zweite Expansionselement und/oder weitere Expansionselemente, wie nachfolgend beschrieben, und/oder ein Einkopplungselement, wie nachfolgend beschrieben, können ein Volumenhologramm und/oder ein Gitter, beispielsweise ein Oberflächengitter, umfassen oder sein.

Das erste Expansionselement und/oder das zweite Expansionselement und/oder weitere Expansionselemente, wie nachfolgend beschrieben, und/oder ein Einkopplungselement, wie nachfolgend beschrieben, können eine Reihenanordnung oder eine Baumstruktur-Anordnung aufweisen.

Unter einer Baumstruktur-Anordnung der betreffenden Elemente wird hierbei verstanden, dass Licht von mindestens einem Element oder einem Bereich eines Elements an mindestens zwei verschiedene Elemente oder Bereiche weitergeleitet wird. Mit anderen Worten kann das Licht verzweigt werden.

Hierbei können verschiedene Verzweigungen bereitgestellt werden, allgemein yⁿ, wobei y die Anzahl der Ausgangslichtbündel oder die Anzahl der Ausgangslichtbündel -1 ist.

Auch eine Kombination verschiedener solcher Anordnungen ist möglich, beispielsweise kann zunächst eine Baumstruktur vorhanden sein und innerhalb eines einzelnen Zweiges der Baumstruktur können dann optische Elemente oder Bereiche wiederum in Reihenstruktur kombiniert sein.

Der Strahlexpander kann ein drittes Expansionselement umfassen, wobei das erste Expansionselement ferner eingerichtet ist, einen weiteren Teil des Lichts als expandiertes Lichts in eine vierte Richtung an das dritte Expansionselement zu leiten, wobei die vierte Richtung von der ersten, zweiten und dritten Richtung verschieden ist und
wobei das dritte Expansionselement eingerichtet ist, den weiteren Teil des expandierten Lichts zu empfangen, entlang der vierten Richtung aufzuweiten und über die Abgabefläche als einen weiteren Teil des zweifach expandierten Lichts in eine fünfte Richtung bereitzustellen, wobei die fünfte Richtung zumindest teilweise nicht in dem Brillenglas verläuft und
wobei das dritte Expansionselement eingerichtet ist, den weiteren Teil des zweifach expandierten Lichts effektiv zu fokussieren.

Durch eine derartige Anordnung kann gewährleistet sein, dass das aktive Augenimplantat in einem größeren Bereich von Blickrichtungen des Benutzers zuverlässig mit Energie versorgt wird.

Hierbei kann die vierte Richtung entgegengesetzt zu der zweiten Richtung sein. Die fünfte Richtung kann der dritten Richtung entsprechen oder nicht senkrecht zu der dritten Richtung sein.

Es kann sein, dass die vierte und zweite Richtung einander im Wesentlichen entsprechen. Beispielsweise kann das erste Expansionselement entlang der ersten Richtung verlaufen und so ausgestaltet sein, dass das zweite und das dritte Expansionselement jeweils mit einem Abstand in der ersten Richtung in der zweiten Richtung vom ersten Expansionselement entfernt sind. Beispielsweise kann das erste Expansionselement länglich in einem oberen Bereich eines Brillenglases ausgeführt sein, und das zweite und dritte Expansionselement jeweils unterhalb des ersten Expansionselements angeordnet sein, wobei das zweite und dritte Expansionselement voneinander beabstandet sein können.

Das zweifach expandierte Licht kann ein konvergentes Lichtbündel mit einem Fokuspunkt umfassen.

Der Fokuspunkt kann zwischen 3 mm und 50 mm von dem Brillenglas entfernt sein. Bevorzugt kann der Fokuspunkt zwischen 10 mm und 30 mm von dem Brillenglas entfernt sein.

Der Fokuspunkt kann somit so liegen, dass der Fokuspunkt näherungsweise im Augendrehpunkt liegt, z. B. weniger als 5 mm von einem Augendrehpunkt des Auges entfernt ist, beispielsweise in Fällen, in denen ein Abstand von der Pupille zum Brillenglas in einem Bereich von ca. 10 mm bis 20 mm gewählt wird und der Abstand von der Pupille zum Augenmittelpunkt, der im Wesentlichen dem Augendrehpunkt entsprechen kann, ca. 9 mm beträgt. Auch kann der Fokuspunkt so liegen, dass er näherungsweise auf der Pupille des Auges liegt, z. B. weniger als 5 mm von einem Zentrum der Pupille entfernt. Der Fokuspunkt kann auch zwischen dem Augendrehpunkt und dem Zentrum der Pupille liegen, beispielsweise weniger als 10 mm von einer gedachten Verbindungslinie entfernt, beispielsweise weniger als 5 mm von einer gedachten Verbindungslinie entfernt.

In Fällen mit anderen anatomischen und/oder vorrichtungsbedingten Gegebenheiten können die Werte entsprechend angepasst werden.

Dies kann für den Fall des Fokus bei dem Zentrum der Pupille den Vorteil haben, dass unabhängig von einer Größe der Pupille möglichst viel des Lichts ins Auge eingekoppelt wird. Wird der Fokuspunkt nahe des Augendrehpunkts gelegt, kann für verschiedene Blickrichtungen eine gleichmäßige Ausleuchtung gewährleistet werden, wenn die Fläche des zweiten Expansionselements groß genug ist. Wird der Fokus entlang der Verbindungslinie positioniert kann ein Kompromiss zwischen den beiden beschriebenen Fällen gewählt werden.

Die Vorrichtung kann eine Steuerung umfassen, wobei die Steuerung eingerichtet sein kann, eine Blickrichtung des Benutzers zu bestimmen. Ferner kann die Vorrichtung eingerichtet sein, die effektive Fokussierung des zweifach expandierten Lichts in Reaktion auf eine Änderung der Blickrichtung des Auges anzupassen.

In den Ausführungsbeispielen, bei denen ein zweites und ein drittes Expansionselement zur Verfügung stehen, kann die Steuerung auch eingerichtet sein, in Reaktion auf eine erkannte Blickrichtung ein Verhältnis der Teile des expandierten Lichts, die jeweils an das zweite und das dritte Expansionselement geleitet werden, zu modifizieren. Hierdurch kann die Energieeffizienz der Vorrichtung verbessert werden.

Die Vorrichtung kann einen Aufnahmevorrichtung und einen Scanspiegel umfassen. Hierbei kann die Steuerung eingerichtet sein, basierend auf einer Information der Aufnahmevorrichtung die Blickrichtung des Benutzers zu bestimmen und basierend auf der Blickrichtung den Scanspiegel anzusteuern.

Die Vorrichtung kann eingerichtet sein, die effektive Fokussierung in eine sechste Richtung zu vergrößern, wobei die sechste Richtung senkrecht zur dritten Richtung sein kann.

Unter einem Vergrößern der Fokussierung wird verstanden, dass zumindest in einer Richtung in der Fokusebene die räumliche Ausdehnung des Lichts vergrößert wird, also das Licht eine größere Fläche in der Fokusebene einnimmt. Dies kann dazu führen, dass für eine feste Lichtenergie in der Fokusebene die Leistungsdichte des Lichtes mit vergrößerter Fokussierung gegenüber der nicht-vergrößerten Fokussierung abnimmt. In anderen Worten kann die Brillanz des Lichtes in der Fokusebene verringert werden. Dies kann zu einer verbesserten Sicherheit der Vorrichtung führen, da verhindert werden kann, dass zu stark konzentrierte Lichtstrahlung von der Vorrichtung abgegeben wird. Dies kann es erlauben, ein Unterschreiten sicherheitsrelevanter Grenzwerte der Bestrahlungsstärke der einzelnen Bereiche des menschlichen Auges durch die Lichtquelle, sowohl für das ausgekoppelte Licht als auch für Strahlexpander-Elemente, zu gewährleisten.

Unter effektiver Fokussierung kann auch verstanden werden, dass eine oder mehrere Strahltaillen definiert werden können. Eine Vergrößerung der der Fokussierung ist dann für mindestens eine Strahltaille eine anisotrope Veränderung der Form der Strahltaille, wobei in einem Querschnitt der Strahltaille, beispielsweise entlang der dritten Richtung, die Ausdehnung der Strahltaille zumindest in eine Richtung, beispielsweise entlang der sechsten Richtung, die in der Querschnittsebene liegen kann und somit senkrecht auf der dritten Richtung stehen kann, vergrößert wird.

Zur Vergrößerung des Fokus kann die Vorrichtung eine Streuscheibe und/oder eine Mikrooptik zwischen der Lichtquelle und einer Kollimationsoptik umfassen, wobei die Streuscheibe und/oder Mikrooptik eingerichtet ist, mehrere Strahlenbündel mit jeweiligem Versatz zueinander zu erzeugen und als das kollimierte Licht bereitzustellen und/oder
- die Lichtquelle eine aktive Beleuchtungsoptik umfasst, die eingerichtet ist, zeitlich veränderliche Strahlenbündel mit jeweiligem Versatz zueinander als das kollimierte Licht bereitzustellen und/oder
- das zweite Expansionselement und/oder die Abgabefläche eingerichtet ist, das zweifach expandierte Licht zu streuen und/oder
- das zweite Expansionselement und/oder die Abgabefläche eingerichtet ist, mehrere entlang der vierten Richtung versetzte Fokusse zu erzeugen.

Diese Mittel zur Vergrößerung des Fokus sind Beispiele dafür, wie die effektive Fokussierung des zweiten Expansionselements beeinflusst werden kann.

Bei manchen optischen Elementen kann eine Streufunktionalität auch direkt durch das optische Element selbst herbeigeführt werden. Beispielsweise kann das zweite Expansionselement eine Streufunktion umfassen, beispielsweise wenn das zweite Expansionselement als ein Volumenhologramm ausgeführt ist. In solchen Fällen kann zusätzlich eine Streuscheibe verwendet werden, um einen kombinierten Effekt zu erzielen. In anderen Fällen kann auf die Streuscheibe verzichtet werden.

Das zweifach expandierte Licht kann mehrere räumlich begrenzte Wellen umfassen. Diese können jeweils von verschiedenen Bereichen der Abgabefläche zumindest teilweise in Richtung der dritten Richtung propagieren, wobei sich die mehreren räumlich begrenzten ebenen Wellen zumindest teilweise kreuzen und/oder divergieren.

Teilweise kreuzen kann auch bedeuten, dass das Kreuzen nur in einer Projektion der räumlich begrenzten ebenen Wellen auf eine Ebene erfolgt, beispielsweise indem die Wellen sich im dreidimensionalen Raum nicht kreuzen, da sie einen Höhenversatz aufweisen. Anders gesagt kann mindestens eine Ebene existieren, die von zwei Vektoren aufgespannt wird, wobei ein Vektor ein Vektor entlang der dritten Richtung ist, so dass die ebenen Wellen, in diese Ebene projiziert, sich kreuzen.

Das zweite Expansionselement kann mehrere Segmente aufweisen und die mehreren Segmente können jeweils eingerichtet sein, jeweils eine der mehreren räumlich begrenzten Wellen bereitzustellen.

Die mehreren Segmente können beispielsweise hexagonal oder rechteckig oder eine Kombination davon sein.

Das erste und/oder zweite Expansionselement kann ein mehrfachbelichtetes Volumenhologramm (manchmal auch als "gemultiplextes" Volumenhologramm bezeichnet) umfassen.

Die mehreren räumlich begrenzten Wellen können mindestens eine oder eine Kombination von: ebenen Wellen, Wellenfronten mit einer konvergenten Wellenfront, Wellenfronten mit einer divergenten Wellenfront sein.

Unter einer Kombination von räumlich begrenzten Wellen wird hierbei verstanden, dass von verschiedenen Ausgangspunkten unterschiedliche Wellenarten ausgehen können. Beispielsweise kann von einer ersten Position eine ebene Welle ausgehen, von einer zweiten Position eine konvergente Wellenfront und von einer dritten Position eine divergente Wellenfront.

In manchen Ausführungsbeispielen kann mindestens eine der räumlich begrenzten Wellen einer zweiten räumlich begrenzten Welle im Wesentlichen entsprechen, jedoch einen räumlichen Versatz entlang der zweiten und/oder ersten Richtung aufweisen.

Im Wesentlichen entsprechen kann bedeuten, dass die Ausbreitungseigenschaften ähnlich oder identisch sind. Beispielsweise kann die Ausbreitungsrichtung im Wesentlichen übereinstimmen, beispielsweise können jeweilige normierte Vektoren entlang der Ausbreitungsrichtung einen Winkel von weniger als 10°, 5°, 1° oder 0,1° zueinander einschließen. Hierbei können die Ausgangspunkte der jeweiligen räumlich begrenzten Wellen identisch oder verschieden sein.

Die Lichtquelle kann eine Laserdiode mit einem Astigmatismus umfassen und das Brillenglas kann eingerichtet sein, eine anisotrope Divergenzanpassung des Lichts von der Laserdiode vorzunehmen.

Das Brillenglas kann auf verschiedene Weisen eingerichtet sein, die anisotrope Divergenzanpassung des Lichts von der Laserdiode vorzunehmen. Beispielsweise kann die Divergenzanpassung durch auf das Brillenglas aufgebrachte Elemente erfolgen. Solche aufgebrachten Elemente können beispielsweise brechende und/oder reflektierende und/oder beugende Flächen sein, beispielsweise Linsen, Spiegel und/oder Oberflächengitter. Zusätzlich oder alternativ kann es auch möglich sein, eingebettete Elemente zu verwenden, beispielsweise Volumenhologramme wie zuvor beschrieben.

Es können auch mehrere Lichtquellen verwendet werden, indem die hier beschriebenen Konzepte mit Segmentierungsansätzen kombiniert werden. Solche Segmentierungen sind für sich genommen aus der WO 2017/180403 A1 bekannt.

Hierbei kann es ebenfalls möglich sein, in dem Brillenglas verschiedene, lichtquellenspezifische Strahlexpander jeweils wie zuvor und nachfolgend beschrieben einzubringen. Die Expansionselemente können hierbei beispielsweise aus unterschiedlichen Richtungen beleuchtet werden. Die dafür nötige Strahltrennung kann entweder bereits an einem Einkopplungselement, welches beispielsweise mittels zwei Umlenkelementen ausgeführt sein kann oder an einem Umlenkelement erfolgen. Hierdurch ist es möglich, bei gleicher Fläche des Brillenglases einen größeren Bereich der Netzhaut auszuleuchten und/oder eine Energieversorgung für einen größeren Rollbereich der Augen sicherzustellen.

Das Brillenglas kann zum Erreichen der anisotropen Divergenzanpassung des Lichts von der Laserdiode ein Einkopplungselement mit anisotropen optischen Eigenschaften umfassen. Das Brillenglas kann aber auch über einen größeren räumlichen Bereich verteilt anisotrope Eigenschaften haben, beispielsweise kann das Brillenglas einen Lichtwellenleiter mit anisotropen Eigenschaften umfassen, wodurch die anisotrope Divergenzanpassung erreicht werden kann.

Das Brillenglas kann einen Wellenleiter und transparentes Material umfassen, wobei der Strahlexpander in oder auf dem Wellenleiter angeordnet ist und das Licht von der Lichtquelle in den Wellenleiter eingekoppelt wird, wobei das transparente Material den Wellenleiter auf mindestens einer Seite zumindest teilweise umgibt.

Sollte der plane Wellenleiter aus Glas gefertigt werden, aber auch in anderen Fällen, kann es aus Sicherheitsgründen, beispielsweise zum Splitterschutz vorteilhaft sein, den Wellenleiter zu kapseln, beispielsweise zwischen Kunststoffschichten, die beispielsweise Polycarbonat umfassen können oder daraus bestehen. Neben einer Kapselung zwischen zwei planen Kunststoffwafern ist beispielsweise auch das Einbringen in ein gewölbtes Kunststoffglas denkbar. Hierdurch kann die Ästhetik der Brille deutlich verbessert werden. Für beide Varianten stehen verschiedenste Fertigungsverfahren zur Verfügung. Beispiele sind:
Ein Befestigen zwischen zwei spritzgegossenen Schalen mit Luftspalt. In diesem Fall erfolgt die Totalreflexion im Wellenleiter gegen Luft, was mit geringeren Totalreflexionswinkeln im Glas führt. Nachteilig können hierbei in manchen Beispielen zusätzliche Reflexionen an den Grenzflächen (Geisterbilder, zusätzliche Reflexe) sein.

Eine andere Möglichkeit ist beispielsweise ein Einkleben unter Luftausschluss. Hierbei kann eine Totalreflexion am Klebstoff auftreten, was zu größeren Totalreflexionswinkeln führen kann. Dies kann weniger Reflexionen und/oder Geisterbilder zur Folge haben. Hierbei können Klebstoffe mit geringer Brechzahl, beispielsweise in der Nähe von n=1.33, verwendet werden, aber auch die Verwendung anderer Klebstoffe ist möglich.

Eine weitere Möglichkeit ist ein Eingießen in Kunststoff. Der Kunststoff kann beispielsweise UVhärtend sein. Das Eingießen kann auf verschiedene Arten durchgeführt werden, beispielsweise zwischen gewölbten Formschalen. Hierbei kann es vorteilhaft sein, einen Wellenleiter mit einer hohen Brechungszahl und hohen Totalreflexionswinkeln zu verwenden. In manchen Ausführungsbeispielen kann eine Entkopplung durchgeführt werden, indem ein planer Wellenleiter zunächst mit einer transparenten Schicht mit einem niedrigen Brechungsindex, z. B. mit einem ausgehärteten entsprechenden Klebstoff, versehen wird.

Das transparente Material kann eine Wölbung auf einer dem Auge des Benutzers zugewandten Seite des zweiten Expansionselements aufweisen, wobei das transparente Material eingerichtet ist, die effektive Fokussierung des Lichts durch Brechung zu modifizieren.

Somit kann eine Fehlsichtigkeitskorrektur für die Sicht durch die Brille möglich sein, beispielsweise durch Verwendung entsprechender sphärischer oder asphärischer Formen auf der dem Auge abgewandten Seite und/oder der dem Auge zugewandten Seite. Gleichzeitig muss die zusätzliche Krümmung auf der dem Auge zugewandten Seite des Brillenglases bereits bei der Auslegung des Strahlexpanders berücksichtigt werden. Dies kann beispielsweise durch eine Anpassung des zweiten Expansionselements geschehen, beispielsweise indem eine stärkere Brechkraft und/oder eine höhere Krümmung der bereitgestellten Wellenfront durch Veränderung der Eigenschaften des zweiten Expansionselements gewählt wird. Dies kann dazu führen, dass eine zerstreuende Wirkung eines Wellenleiters zumindest teilweise kompensiert werden kann.

Die Vorrichtung kann eingerichtet sein, das Brillenglas mit einem Fassungsscheibenwinkel und einem Vorneigungswinkel in Bezug auf eine Geradeausblickrichtung des Benutzers zu positionieren.

Unter einem Vorneigungswinkel, auch pantoskopischer Winkel wird der Winkel in der Vertikalebene zwischen der Normale zur Vorderfläche eines Brillenglases in dessen Mittelpunkt nach Kastensystem und der Fixierlinie des Auges in Primärposition, die üblicherweise als horizontal angenommen wird, verstanden.

Unter einem Fassungsscheibenwinkel wird ein Winkel zwischen der Fassungsebene und der rechten bzw. linken Scheibenebene verstanden. Der Fassungsscheibenwinkel wird manchmal auch als Wrap-Winkel bezeichnet.

Das Brillenglas kann ein Einkopplungselement und einen damit verbunden Lichtwellenleiter umfassen, wobei der Lichtwellenleiter zum Weitergeben des Lichtes an das erste Expansionselement eingerichtet ist.

Die Lichtquelle kann mindestens eines der folgenden Elemente umfassen:
zwei Einzellichtquellen, die eingerichtet sind Licht in unterschiedlichen Richtungen und/oder in unterschiedlichen Wellenlängenbereichen und/oder an unterschiedlichen Beleuchtungspositionen mindestens eines Einkopplungselements zum Einkoppeln des Lichts in das Brillenglas bereitzustellen,
einen Strahlteiler,
einen Scanspiegel,
ein schaltbares Element.

Auf diese Weise kann zwischen verschiedenen Leuchtverteilungen des Lichts, beispielsweise einer Leuchtverteilung zum Versorgen des Augenimplantats mit Energie und eine Leuchtverteilung für andere Zwecke, oder zwei verschiedenen Leuchtverteilungen zum Versorgen des Augenimplantats mit Energie, umgeschaltet werden, indem die Einzellichtquellen, der Strahlteiler, der Scanspiegel und/oder das schaltbare Element entsprechend angesteuert werden.

Bevorzugt erfolgt dabei das Umschalten in Abhängigkeit von einer Blickrichtung des Auges, die beispielsweise von einem Eyetracker erfasst wird. So kann das Augenimplantat bei der jeweiligen Blickrichtung effizient mit Energie versorgt werden. Hierdurch kann auch eine Versorgung des Augenimplantats über einen großen Blickwinkelbereich (field of view) erfolgen.

Das Brillenglas kann eine Aussparung aufweisen. Hierdurch können manche Untersuchungen des Auges bei getragener Brille ermöglicht werden.

Gemäß einem zweiten Aspekt wird ein Verfahren zum Herstellen einer nutzerspezifischen Brille bereitgestellt, wobei das Verfahren umfasst:
Empfangen von Kopfgeometrieinformationen des Benutzers,
Herstellen einer Vorrichtung gemäß einem der vorherigen Ansprüche basierend auf den Kopfgeometrieinformationen.

Kopfgeometrieinformationen können hierbei Informationen bezüglich der räumlichen Anordnung von anatomischen Merkmalen umfassen oder sein, z. B. Lage des Auges, Lage von Auflagepunkten für Brille, etc. wie diese für sich genommen beispielsweise aus EP 3 410 178 A1 bekannt sind.

Die Vorrichtung kann eine Vorrichtung wie zuvor beschrieben sein, bei der das Brillenglas ein Einkopplungselement und einen damit verbunden Lichtwellenleiter umfasst, wobei der Lichtwellenleiter zum Weitergeben des Lichtes an das erste Expansionselement eingerichtet ist.

Das Verfahren kann ferner ein Aktivieren eines Teils des Brillenglases umfassen, um das zuvor beschriebene Einkopplungselement und/oder ein anderes Einkopplungselement bereitzustellen.

Ein solches Aktivieren ist ein nutzerspezifisches Festlegen von Größe und/oder Eigenschaften des Einkopplungselements.

Solche Verfahren können beispielsweise dann angewendet werden, wenn optisch wirksame Elemente, beispielsweise diffraktive Elemente, beispielsweise Volumenhologramme, im Rahmen des Verfahrens erzeugt werden. Beispielsweise kann es mittels Belichtung möglich sein, diffraktive Elemente in einem dafür vorgesehenen Brillenglas lokal zu erzeugen. Ein solches Belichten kann mittels verschiedener Prozesse durchgeführt werden, beispielsweise mittels Laserschreiben, aber auch andere Belichtungsverfahren sind möglich.

Ein solches Aktivieren kann basierend auf den Kopfgeometrieinformationen erfolgen.

Solche durch Aktivieren erzeugten Einkopplungselemente können, wie oben erwähnt, beispielsweise Volumenhologramme sein. Hierfür können die Brillengläser eine Schichtstruktur aufweisen, die geeignet ist, aktiviert zu werden und sich über eine Bandbreite von möglichen Positionen für das Einkopplungselement erstreckt. In solchen Beispielen kann das Aktivieren ein Belichten umfassen.

Hierdurch kann eine nutzerspezifische Anpassung von serienfertigbaren Brillengläsern, die für eine Vielzahl von Nutzern hergestellt werden können, vorgenommen werden.

Das Verfahren kann ferner umfassen:
Bestimmen eines nutzerspezifischen Lateralversatzes für das Einkopplungselement an dem Brillenglas, und/oder
das Einkopplungselement kann ein Einkopplungsprisma umfassen und das Verfahren kann ein Anpassen einer Position des Einkopplungsprismas umfassen, und/oder mindestens zwei Elemente aus der Gruppe von Einkopplungselementen, dem ersten Expansionselement und dem zweiten Expansionselement können auf mindestens zwei verschiedenen Wafern aufgebracht sein und das Verfahren kann ferner umfassen:
   Bonden der zwei verschiedenen Wafer miteinander in einer Relativposition zueinander, wobei die Relativposition basierend auf den Kopfgeometrieinformationen bestimmt wird.

Das Einkopplungselement kann ein Oberflächengitter und/oder ein Volumenhologramm umfassen, wobei das Einkopplungselement für einen Parameterbereich von Kopfgeometrieinformationen eingerichtet ist, und eine Einkopplungsfläche aufweist, die größer ist als eine Lichtquelleneinkopplungsfläche der Lichtquelle auf dem Einkopplungselement.

Das Verfahren kann ein Positionieren der Lichtquelle basierend auf den Kopfgeometrieinformationen umfassen, wodurch die Lichtquelleneinkopplungsfläche in Bezug zur Einkopplungsfläche fest angeordnet wird.

In manchen Ausführungsbeispielen umfasst das Verfahren weiter:
Deaktivieren eines Teils der Einkopplungsfläche, wobei das Deaktivieren mit einer oder mehreren der folgenden Schritte erfolgt:
- elektrisches beeinflussen eines elektrischen Gitters in der Einkopplungsfläche,
- Abtragen eines UV-Lacks in einem Bereich der Einkopplungsfläche,
- lokales Einbringen eines Materials in ein Oberflächengitter, wobei das Material eine ähnliche Brechzahl wie ein Material des Einkopplungselement aufweist,
- Zerstören einer Wirkung eines Teils eines Hologramms mittels Strahlung, insbesondere elektromagnetische Strahlung und/oder Temperatur.

Hierbei kann der UV-Lack durch entsprechende Belichtung und Entwicklung so gestaltet sein, dass der UV-Lack diffraktive Elemente, beispielsweise Gitterstrukturen, beispielsweise Volumenhologramme, aufweist. Solche diffraktiven Elemente können dann durch lokales Abtragen räumlich selektiv deaktiviert werden, aber auch andere Arten des Deaktivierens sind möglich, wie zuvor und nachfolgend beschrieben.

So können beispielweise diffraktive Strukturen, beispielsweise Hologramme, beispielsweise Volumenhologramme, mittels Strahlung lokal deaktiviert werden, so dass die Wirkung lokal zerstört ist. Alternativ oder ergänzend kann eine Deaktivierung auch mittels Wärmeeintrag, beispielsweise durch lokale, in manchen Beispielen zeitlich begrenzte, Veränderung der Temperatur der diffraktiven Strukturen, erfolgen.

Zusätzlich oder alternativ kann das Verfahren auch mit den zuvor beschriebenen Verfahren kombiniert werden. Beispielsweise kann ein erster Bereich des Brillenglases, wie zuvor beschrieben, aktiviert werden, und ein zweiter Bereich des Brillenglases deaktiviert werden.

Das gesamte Brillenglas oder eine Teilregion des Brillenglases kann als eine Einkopplungsfläche aktiviert werden. Die Teilregion kann hierbei so gewählt werden, dass für eine große Bandbreite von nutzerspezifischen Kopfgeometrieinformationen eine geeignete Position eines Einkopplungselements jeweils in die Teilregion fällt. Ein Brillenglas mit einer aktivierten Teilregion kann auch als Ausgangsmaterial für das Verfahren gewählt werden. In diesem Fall kann der Schritt des Aktivierens im Verfahren entfallen und/oder in den Herstellungsprozess des Brillenglases vorverlagert werden. Dies kann den Vorteil haben, dass die Brillengläser günstig hergestellt werden können, beispielsweise indem Schichtstrukturen auf das Brillenglas in der Herstellung auf einem größeren Bereich aufgebracht werden als bei den jeweiligen angepassten Brillengläsern als Einkopplungsfläche benötigt werden.

Das Einkopplungselement kann nun nutzerspezifisch dadurch bereitgestellt werden, dass der zweite Bereich, der eine Teilmenge des ersten Bereichs sein kann, deaktiviert wird. Der verbleibende Teil des ersten Bereichs bleibt in solchen Fällen aktiviert und kann, teilweise oder vollständig, als Einkopplungselement verwendet werden.

Mit anderen Worten kann das Einkopplungselement durch einen Aktivierungsprozess individualisiert bereitgestellt werden und/oder durch selektives Deaktivieren eines aktiven Bereichs bereitgestellt werden, vergleichbar mit einem positiv- bzw. negativ-Prozess. Auch eine Kombination solcher Prozesse ist möglich, beispielsweise in verschiedenen Bereichen, bei denen ein erster Teil eines Einkopplungselements durch einen positiv- und ein zweiter Teil eines Einkopplungselements durch einen Negativprozess bereitgestellt wird.

Das Deaktivieren kann in einem Deaktivieren eines Teils der Einkopplungsfläche, wie zuvor beschrieben, bestehen oder diesen Prozess umfassen.

Dies kann den Vorteil haben, dass eine Anpassung der Vorrichtung an individuelle Anforderungen und Kopfgeometrien eines Benutzers stark vereinfacht werden kann, beispielsweise indem für Benutzergruppen gleiche Ausgangsformen bereitgestellt werden und mit den o.g. Verfahren eine Anpassung der Vorrichtungen an das Individuum vorgenommen wird.

Auch kann es möglich sein, dass solche Anpassungen nicht durch Anpassung eines einzelnen Elements der Vorrichtung vorgenommen werden, sondern durch eine Kombination verschiedener Elemente, also beispielsweise durch eine Modifikation des ersten Expansionselements und einer Position der Lichtquelle. Aber auch andere Kombinationen sind möglich.

Ein erstes und ein zweites Material haben dann eine ähnliche Brechzahl, wenn sich die jeweiligen Brechzahlen des ersten und zweiten Materials beispielsweise um weniger als 50 %, weniger als 20 %, weniger als 10 %, weniger als 1 % unterscheiden.

Eine Kombination von Aktivieren und Deaktivieren kann hierbei den Vorteil bieten, dass die Kosten für die Herstellung gesenkt werden können.

Das Verfahren kann ferner umfassen:
Empfangen von benötigten Sehhilfe-Eigenschaften für den Benutzer,
Anpassen der effektiven Fokussierung der Vorrichtung basierend auf den benötigten Sehhilfeeigenschaften.

Die effektive Fokussierung der Vorrichtung kann in den Beispielen, in denen die Vorrichtung eine Wölbung aufweist, beispielsweise dadurch erreicht werden, dass die Wölbung basierend auf den benötigten Sehhilfeeigenschaften gewählt wird und basierend auf der Wölbung die Eigenschaften des Pupillenreplikationssystems angepasst werden. Hierdurch kann es möglich sein, die Strahlführung des Lichts weitestgehend unabhängig von den Lichtbrechungseigenschaften der Vorrichtung in einem sichtbaren Wellenlängenbereich zu optimieren.

Durch die beschriebenen Verfahren kann eine an die individuellen Anforderungen eines Benutzers angepasste Vorrichtung bereitgestellt werden, die einen gesteigerten Tragekomfort und eine verbesserte Ästhetik bieten kann.

Die Erfindung wird nachfolgend unter Bezugnahme auf die beigefügte Zeichnung anhand von Ausführungsbeispielen detailliert erläutert. Es zeigen:
Fig. 1 ein Diagramm zur Verdeutlichung von Winkeln eines Brillenglases, wenn diese von einem Benutzer getragen werden,
Fig. 2 eine Brille mit verschiedenen Ausführungsbeispielen für linkes und rechtes Brillenglas,
Fig. 3A und Fig. 3B Querschnittsansichten verschiedener Ausführungsbeispiele mit unterschiedlichen effektiven Fokussierungen,
Fig. 4A und Fig. 4B weiteres Ausführungsbeispiel mit einer alternativen effektiven Fokussierung mittels mehrerer räumlich begrenzter Wellen,
Fig. 5 ein weiteres Ausführungsbeispiel mit einem von transparentem Material umgebenen Wellenleiter,
Fig. 6 ein Ablaufdiagramm für ein Verfahren gemäß verschiedener Ausführungsbeispiele.
Fig. 7 ist ein Diagramm zur Veranschaulichung einer Baumstruktur bei manchen Ausführungsbeispielen.
Fig.8 zeigt Vorrichtungen gemäß verschiedener Ausführungsbeispiele, die mehrkanalig und/oder schaltbar sind.
Fig. 9A zeigt eine Seitenansicht einer Vorrichtung, die eine Untersuchungsmodalität ermöglicht.
Fig. 9B zeigt eine Frontalansicht der Vorrichtung der Fig. 9A.
Fig. 10 zeigt ein weiteres Ausführungsbeispiel einer Vorrichtung, die eine Untersuchungsmodalität ermöglicht.

Im Folgenden werden verschiedene Ausführungsbeispiele detailliert erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente oder Komponenten, weniger Elemente oder Komponenten oder auch zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein.

Zur Vermeidung von Wiederholungen werden gleiche oder einander entsprechende Elemente in verschiedenen Figuren mit dem gleichen Bezugszeichen bezeichnet und werden nicht mehrmals erläutert.

Die Figuren zielen auf die Darstellung der zugrundeliegenden Prinzipien ab. Beispielsweise Oberflächenformen und Brechungen können daher schematisch angedeutet sein. Brechungen können beispielsweise übertrieben dargestellt oder vernachlässigt werden.

Bei den folgenden Ausführungsbeispielen wird ein Retinaimplantat als Beispiel für ein aktives Augenimplantat verwendet. Die beschriebenen Techniken sind jedoch auch auf andere aktive Augenimplantate, beispielsweise die eingangs erwähnten Augenimplantate, anwendbar.

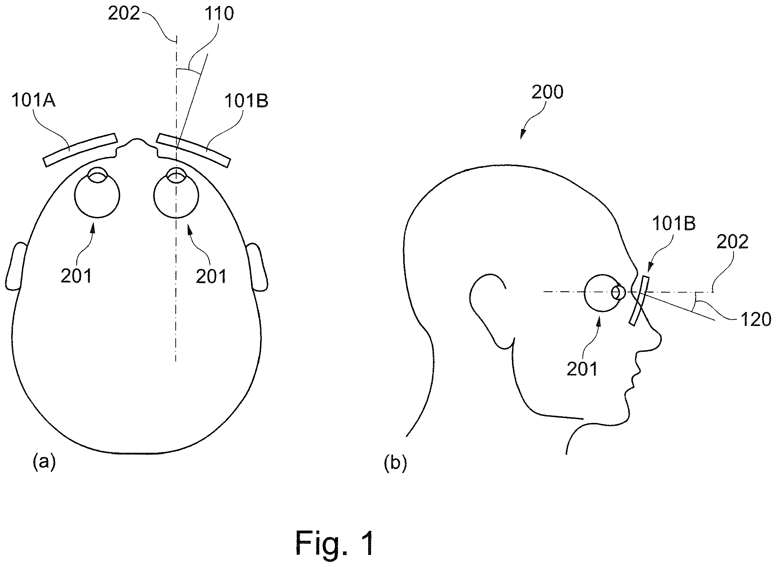

Fig. 1 zeigt eine Skizze zur Verdeutlichung von Winkeln eines Brillenglases, wenn diese von einem Benutzer getragen werden.

Eine Vorrichtung zur Versorgung eines aktiven Augenimplantats in einem Auge 201 eines Benutzers 200 mit Energie kann als ein kopfgetragenes, brillenähnliches System ausgeführt sein und mindestens ein Brillenglas umfassen. Im gezeigten Beispiel der Fig. 1 umfasst die Vorrichtung zwei Brillengläser 101A, 101B. Fig. 1 (a) zeigt eine Aufsicht, Fig. 1 (b) eine Seitenansicht eines Benutzers 200 der Vorrichtung. Die Brillengläser 101A, 101B können verschiedene Winkel relativ zur Geradeausblickrichtung 202 des Benutzers 200 aufweisen. Wie in Fig. 1 gezeigt, kann die Vorrichtung so ausgeführt sein, dass sich ein Fassungsscheibenwinkel 110 und ein Vorneigungswinkel 120, wie zuvor beschrieben, ergibt. Der Vorneigungswinkel 120 kann beispielsweise zwischen 5° und 20° sein und der Fassungsscheibenwinkel 110 kann beispielsweise zwischen 5° und 20° liegen. Solche Winkel ungleich 0° können die Vorrichtung ästhetisch vorteilhaft machen.

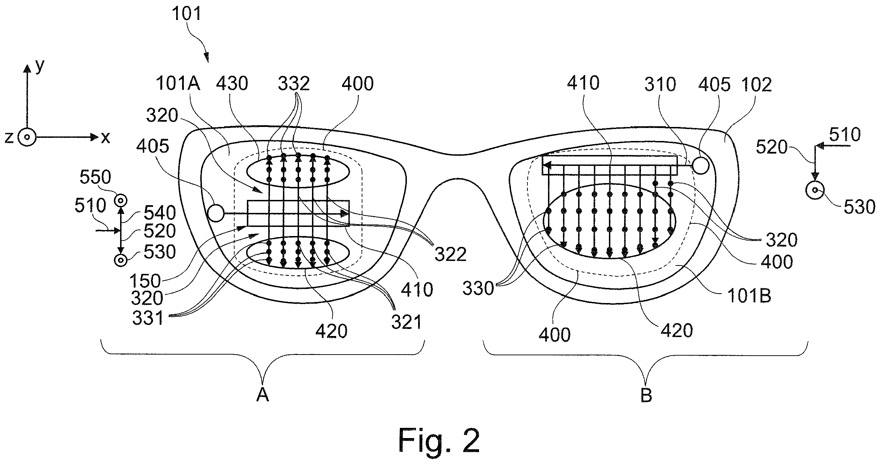

Fig. 2 zeigt ein Ausführungsbeispiel einer Vorrichtung zur Versorgung eines aktiven Augenimplantats in einem Auge eines Benutzers mit Energie. Die Vorrichtung ist in diesem Ausführungsbeispiel als eine Brille 101 ausgeführt, die zwei Brillengläser 101A und 101B umfasst, die in einem Brillengestell 102 angeordnet sind. Die Vorrichtung des Ausführungsbeispiels der Fig. 2 umfasst jeweils eine Vorrichtung A zur Energieversorgung eines aktiven Augenimplantats im linken Auge und eine Vorrichtung B zur Energieversorgung eines aktiven Augenimplantats im rechten Auge. Hierbei ist zu beachten, dass weitere Ausführungsbeispiele auch nur eine der beiden Vorrichtungen A oder B umfassen können, beispielsweise als Monokel ausgeführt sein können oder eine Vorrichtung, beispielsweise die Vorrichtung A, umfassen können und als reguläre Brille ausgeführt sein können, so dass in diesem Beispiel das Brillenglas 101B dann keinen Strahlexpander umfassen würde, beispielsweise wenn bei dem Benutzer nur in einem Auge ein aktives Augenimplantat vorhanden ist.

Im gezeigten Beispiel wird von der Brille 101 die Energieversorgung sowohl für ein aktives Augenimplantat im linken als auch im rechten Auge bereitgestellt. In solchen Fällen, in denen die Brille 101 Vorrichtungen A, B für beide Augen bereitstellt, können die Vorrichtungen für linkes Auge A und rechtes Auge B identisch oder ähnlich, beispielsweise spiegelsymmetrisch, sein. Sie können aber auch unterschiedlich ausgeführt sein, wie im Beispiel der Fig. 2 gezeigt. Im gezeigten Beispiel der Vorrichtung 100 umfasst die Vorrichtung eine Vorrichtung A für das linke Auge aus Patientensicht und eine zweite Vorrichtung B für das rechte Auge aus Patientensicht. Die Brillengläser 101A, 101B der Vorrichtungen A, B können hierbei Winkel relativ zur Geradeausblickrichtung aufweisen, wie im Zusammenhang mit Fig. 1 bereits erläutert.

Die Vorrichtung B zeigt ein erstes Ausführungsbeispiel für eine Vorrichtung mit einem Brillenglas 101B zur Versorgung eines aktiven Augenimplantats in einem rechten Auge eines Benutzers mit Energie. Die Vorrichtung umfasst eine nicht gezeigte Lichtquelle, wobei Licht von der Lichtquelle an einem Einkopplungselement 405 bereitgestellt wird. Hierbei ist zu bemerken, dass in manchen Ausführungsbeispielen ein Einkopplungselement 405 nicht vorhanden ist, sondern an dieser Stelle direkt eine Lichtquelle eingearbeitet sein kann. Auch kann es möglich sein, dass sich eine oder mehrere Lichtquellen in dem Brillengestell 102 befinden.

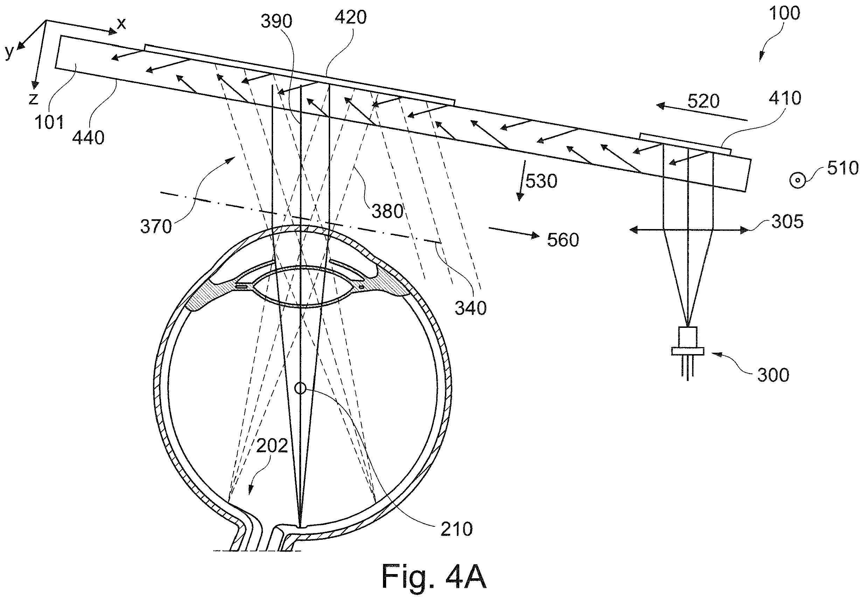

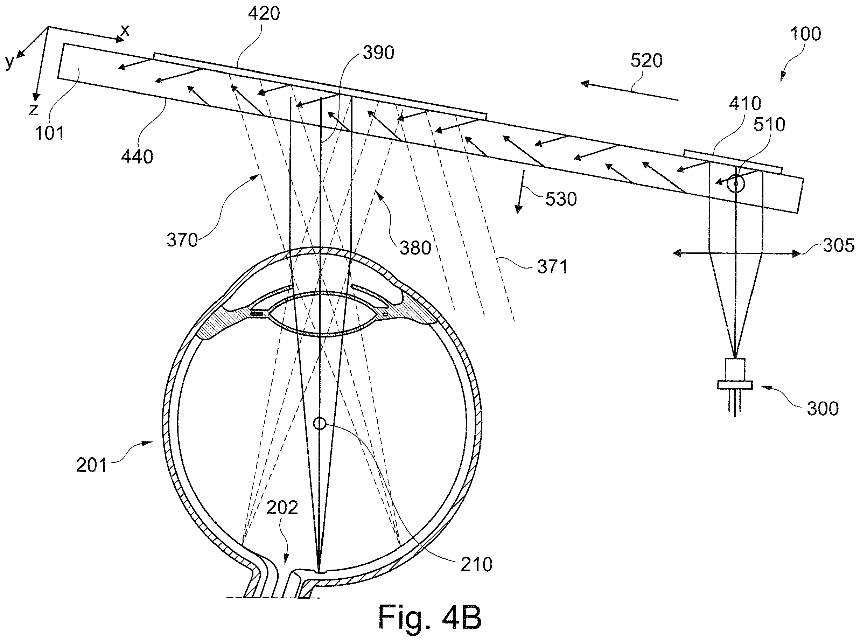

Die Vorrichtung weist einen Strahlexpander 400, der im Beispiel der Fig. 4 in dem Brillenglas 101 angeordnet ist. Der Strahlexpander 400 umfasst ein erstes Expansionselement 410 und ein zweites Expansionselement 420.

Die Vorrichtung B ist eingerichtet, Licht 310 von der Lichtquelle in das Brillenglas 101 einzukoppeln und zu dem ersten Expansionselement 410 zu leiten. Das erste Expansionselement 410 ist eingerichtet, das Licht 310 zu empfangen, entlang einer ersten Richtung 510 aufzuweiten und als expandiertes Licht 320 in einer zweiten Richtung 520 an das zweite Expansionselement 420 zu leiten.

Dies kann den Vorteil bieten, dass die Designfreiheitsgrade bei der Erstellung solcher Pupillenexpansionselemente es ermöglichen, die Beleuchtungsprofile der Vorrichtung den Anforderungen der Energieempfangsvorrichtungen der aktiven Augenimplantate anzupassen.

Je nach Art des aktiven Augenimplantats kann beispielsweise ein homogenes Beleuchtungsmuster oder ein anderes, an die Empfängergeometrie und -anforderungen angepasstes Intensitätsverteilungsmuster erstrebenswert sein und durch Pupillenexpansionssysteme bereitgestellt werden.

Bei der Darstellung der Fig. 2 ist hierbei zu beachten, dass die jeweiligen Richtungen für die Ausführungsbeispiele A und B unterschiedlich (im gezeigten Beispiel spiegelsymmetrisch) gewählt sind. Hieraus ist zu ersehen, dass die konkreten Richtungen nur als Beispiel zu verstehen sind. Wäre beispielsweise das Einkopplungselement 405 der Vorrichtung B anstelle der gezeigten Position rechts oben links unten angebracht, würden sich die Richtungen entsprechend umkehren, so dass eine gleiche Expansionswirkung erzielt werden kann.

Die zweite Richtung 520 kann von der ersten Richtung 510 verschieden sein, im gezeigten Beispiel der Vorrichtung B ist dieser Unterschied 90°.

Das zweite Expansionselement 420 ist eingerichtet, das expandierte Licht 320 zu empfangen, entlang der zweiten Richtung 520 aufzuweiten und über eine Abgabefläche 440 als zweifach expandiertes Licht 330 in eine dritte Richtung 530 bereitzustellen, wobei die dritte Richtung 530 zumindest teilweise nicht in dem Brillenglas 101 verläuft. In den gezeigten Beispielen der Vorrichtung A und B der Fig. 2 verläuft die dritte Richtung 520 allgemein aus der Zeichenebene auf den Betrachter hin hinaus, so dass bei den Vorrichtungen A und B das Licht 530 jeweils zu dem jeweiligen Auges des Benutzers hin geleitet wird. In dem Beispiel der Fig. 2 sind diskrete Auskoppelungspunkte für jeweilige Lichtstrahlen dargestellt, es kann aber auch möglich sein, dass eine kontinuierliche Auskoppelung entlang der Fläche verläuft oder eine Kombination von Bereichen, in denen eine flächige und eine diskrete Auskoppelung erfolgt, bzw. an denen der Anteil des ausgekoppelten Lichts verglichen mit dem Anteil des weitergeleiteten Lichts örtlich variiert. Hierdurch kann insbesondere eine gleichmäßige Flächenlichtintensität erreicht werden. In anderen Ausführungsbeispielen können aber auch bewusst ungleichmäßige Ausleuchtungsprofile herbeigeführt werden, beispielsweise um bestimmten Anforderungen eines aktiven Augenimplantats gerecht werden zu können.

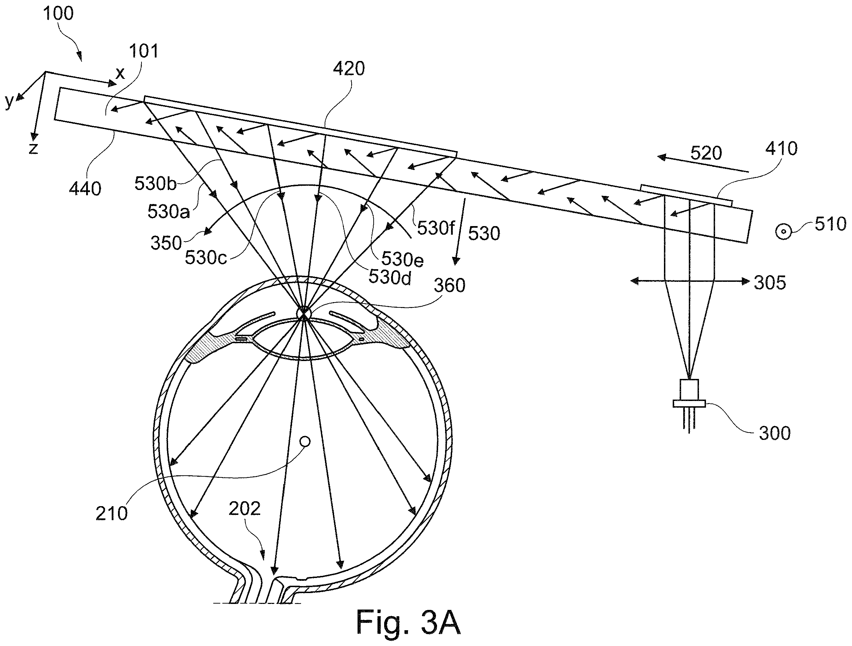

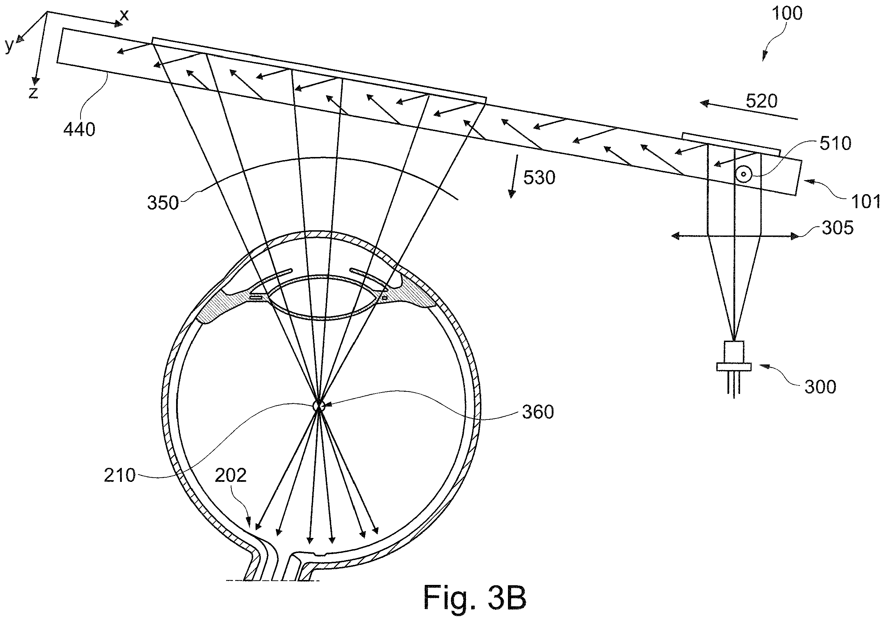

Das zweite Expansionselement 530 ist eingerichtet, das zweifach expandierte Licht 330 effektiv zu fokussieren. Dies wird im Zusammenhang mit den Figuren 3A, 3B bis 5 genauer erläutert.

Die Vorrichtung A zeigt ein anderes Beispiel. Die Vorrichtung A ist an die Vorrichtung B angelehnt, wobei gleiche Bezugszeichen gleiche Elemente bezeichnen. Die jeweiligen Eigenschaften und Richtungen 510-530 können jedoch von dem Ausführungsbeispiel der Vorrichtung B abweichen.

Zusätzlich weist der Strahlexpander 400 der Vorrichtung A gegenüber dem zuvor beschriebenen Strahlexpander 400 der Vorrichtung B ein drittes Expansionselement 430 auf.

Bei der Vorrichtung B ist das Expansionselement 410 eingerichtet, das expandierte Licht in die zweite Richtung 520 bereitzustellen, wie zuvor erläutert. Das Expansionselement 410 der Vorrichtung A ist eingerichtet, das Licht zu expandieren und aufzuteilen. Einen Teil des expandierten Licht 320 stellt das Expansionselement 410 in die zweite Richtung 520 bereit. Einen weiteren Teil des expandierten Lichts 320 leitet das das erste Expansionselement 410 der Vorrichtung A in einer vierten Richtung 540 an das dritte Expansionselement 430. Die vierte Richtung ist hierbei von der ersten, zweiten und dritten Richtung verschieden. Im gezeigten Beispiel ist die vierte Richtung 540 entgegengesetzt zur dritten Richtung 530.

Das dritte Expansionselement 430 ist eingerichtet, das expandierte Licht 320, bzw. den weiteren Teil des expandierten Lichts 322 zu empfangen, entlang der vierten Richtung 540 aufzuweiten und über die Abgabefläche 430 als einen weiteren Teil des zweifach expandierten Lichts in eine fünfte Richtung 550 bereitzustellen, wobei die fünfte Richtung 550 zumindest teilweise nicht in dem Brillenglas verläuft.

Das zweifach expandierten Lichts 330 umfasst entsprechend einen Teil 331 und den weiteren Teil 332, wobei der Teil 331 des zweifach expandierten Lichts das von dem zweiten Expansionselement 420 bereitgestellte zweifach expandierte Licht 330 ist.

Das dritte Expansionselement 430 ist eingerichtet, den weiteren Teil des zweifach expandierten Lichts 332 effektiv zu fokussieren.

Im gezeigten Beispiel der Vorrichtung A sind dritte Richtung 530 und fünfte Richtung 550 identisch. Dies muss aber nicht notwendigerweise der Fall sein. Beispielsweise kann es möglich sein, dass die fünfte Richtung 550 eine Richtungskomponente in negativer y-Richtung aufweist und die dritte Richtung eine Richtungskomponente in positiver y-Richtung aufweist. Hierdurch kann es möglich sein, dass auch die Positionierung von drittem und viertem Expansionselement zur effektiven Fokussierung des zweifach expandierten Lichts 331, 332, 330 beitragen kann.

Auch kann es möglich sein, dass die jeweiligen Richtungen Schwerpunktrichtungen darstellen, also beispielsweise einen Mittelwert der Richtungen beschreiben, mit denen Licht aus den Expansionsvorrichtungen ausgekoppelt wird. Hierdurch kann es beispielsweise möglich sein, dass das zweifach expandierte Licht, welches von der dritten Expansionsvorrichtung 430 bereitgestellt wird in einem Punkt fokussiert wird. Aber auch andere Strahlengänge des zweifach expandierten Lichtes sind möglich. Dies wird nachfolgend anhand von Beispielen weiter erläutert.

Die Figuren 3A und 3B sowie 4A und 4B zeigen hierzu Querschnittsansichten verschiedener Ausführungsbeispiele mit unterschiedlichen effektiven Fokussierungen.

In den Figuren 3A, 3B, 4A, 4B und 5 ist schematisch eine Lichtquelle 300, die beispielsweise eine Infrarotlichtquelle sein kann, gezeigt. Die Vorrichtung kann ferner ein oder mehrere optische Elemente zwischen der Lichtquelle 300 und dem Einkopplungselement 405 umfassen. Im gezeigten Beispiel ist eine Kollimationsoptik 305 schematisch angedeutet, aber auch eine Kombination mit anderen optischen Elementen, beispielsweise mit den zuvor erwähnten Streuscheiben, ist möglich. Auch Umlenkeinheiten können bereitgestellt werden, so dass das Licht von einem Einkopplungselement (nicht gezeigt) in die erste Richtung 510 propagiert und von dem ersten 410 und zweiten 420 Expansionselement als zweifach expandiertes Licht in die dritte Richtung 530 bereitgestellt wird. Die Figuren 3A, 3B, 4A und 4B zeigen verschiedene Varianten, wie die erwähnte effektive Fokussierung erreicht werden kann.

Unter effektiver Fokussierung wird hierbei verstanden, dass das zweifach expandierte Licht 330 auf eine gedachte Fokusfläche 340 zwischen Abgabefläche 440 und dem aktiven Augenimplantat 202 entlang der dritten Richtung 530 gelenkt wird, wobei die gedachte Fokusfläche 340 kleiner als die Abgabefläche 440 ist.

Verschiedene Beispiele sind hierfür in den Figuren gezeigt. In der Fig. 3A erzeugt das zweite Expansionselement eine Lichtverteilung, die so eingestellt ist, dass das durch die Abgabefläche 440 abgegebene zweifach expandierte Licht ein konvergentes Strahlenbündel darstellt, dessen Fokuspunkt 360 in der Augenpupille liegt. Hierbei ist zu beachten, dass die zuvor und nachfolgend beschriebenen Richtungen effektive Richtungen darstellen. Beispielsweise erfolgt die Ausbreitung in die zweite Richtung 520, wobei aber einzelne Lichtbündel in Totalreflexion entlang des Brillenglases reflektiert werden und daher, wie von den Pfeilen angedeutet, auf kurzen Skalen in andere Richtungen als die zweite Richtung 520 propagieren können. Ebenfalls wird das zweifach expandierte Licht in die dritte Richtung 530 bereitgestellt, einzelne Lichtbündel können aber hierfür unterschiedliche Richtungen 530a-f aufweisen. Gleiches gilt für die anderen Beispiele entsprechend.

In manchen Ausführungsbeispielen der Fig. 3A kann die Lage des Fokuspunktes in der Pupille dazu führen, dass der Fokus bei Rotation des Auges statisch bleibt und bei extremen Blickrichtungsänderungen die Pupille verfehlen könnte.

Die Vorrichtung 100 der Fig. 3B entspricht im Wesentlichen der Vorrichtung 100 der Fig. 3A, allerdings ist hier ein anderer Fokuspunkt 360 gewählt, so dass der Fokuspunkt 360 nicht im Bereich der Pupille, sondern im Bereich des Augendrehpunkts 210 liegt. Nach Propagation durch die Augenpupille fächert sich das Bündel somit wieder auf und beleuchtet ein ausgedehntes Netzhautareal.

Dies führt bei den dargestellten Ausführungsbeispielen dazu, dass die Ausleuchtung weniger stark von der Blickrichtung des Benutzers abhängig ist. Ein Vorteil kann hierbei sein, dass auch bei Augendrehung ein konstant großes Netzhautareal ausgeleuchtet werden kann. In manchen Ausführungsbeispielen kann dieser Fokuspunkt 360 jedoch dazu führen, dass nur ein Teil des abgestrahlten Lichtes, welches als ein konvergentes Bündel bereitgestellt wird, bei einer gegebenen Blickrichtung zu einem aktiven Augenimplantat 202 gelangt, was die Energiebilanz der Vorrichtung 100 verschlechtern kann.

Eine alternative Option besteht daher darin, die Blickrichtung des Benutzers, beispielsweise mittels Eye Tracking, zu messen und den Fokus des konvergenten Bündels aktiv nachzuführen, z. B. indem durch einen Scanspiegel zwischen der Lichtquelle 300 und dem Brillenglas 101 die Richtung des eingekoppelten Strahls modifiziert wird.

Die Fokussierung in einem Punkt kann in manchen Ausführungsbeispielen und je nach Energieanforderungen des aktiven Augenimplantats dazu führen, dass in der Nähe des Fokus der Strahlen eine hohe Leistungsdichte auftreten kann, die die gesetzlichen Grenzen überschreiten kann. Um dem entgegen zu wirken, kann der Fokus lateral, beispielsweise in eine sechste Richtung 560, vergrößert werden. Dies kann entweder mit einer Vorrichtung gemäß der Fig. 3A und Fig. 3B durch einbringen von Streuscheiben oder Mikrooptiken, wie zuvor beschrieben, erreicht werden. Durch solche optischen Elemente können jeweilige Fokusse an unterschiedliche laterale Positionen abgelenkt werden. Der Bereich der Fokusse kann hierbei homogen oder diskret ausgeleuchtet werden.

Bei manchen optischen Elementen kann eine Streufunktionalität auch direkt durch das optische Element selbst herbeigeführt werden. Beispielsweise kann das zweite Expansionselement eine Streufunktion umfassen, beispielsweise wenn das zweite Expansionselement als ein Volumenhologramm ausgeführt ist.

Eine andere Möglichkeit wird nachfolgend im Zusammenhang mit den Fig. 4A und Fig. 4B beschrieben.

Fig. 4A und Fig. 4B zeigen alternative effektive Fokussierungen mittels mehrerer räumlich begrenzter Wellen.

Während die Vorrichtungen 100 der Fig. 3A und Fig. 3B konvergente Lichtbündel 350 als zweifach expandiertes Licht bereitstellen, ist die Vorrichtung 100 der Fig. 4A und Fig. 4B eingerichtet, eine effektive Fokussierung des zweifach expandierten Lichts dadurch zu erreichen, dass das zweifach expandierte Licht 330 mehrere räumlich begrenzte Wellen 370, 380, 390 umfasst, die jeweils von verschiedenen Bereichen der Abgabefläche zumindest teilweise in Richtung der dritten Richtung 530 propagieren, wobei sich die mehreren räumlich begrenzten ebenen Wellen zumindest teilweise kreuzen und/oder divergieren. Die effektive Fokussierung wird somit durch die räumliche Anordnung der ebenen Wellen erreicht.

In den Beispielen der Fig. 4A und Fig. 4B sind ebene Wellen gezeigt, aber auch andere Wellenformen sind möglich, beispielsweise ebenen Wellen, Wellenfronten mit einer konvergenten Wellenfront oder Wellenfronten mit einer divergenten Wellenfront.

Dies ist kann beispielsweise erreicht werden, indem das Auskoppelelement segmentiert wird und pro Segment ein Hologramm mit unterschiedlicher Periode und Orientierung eingebracht wird. Die Segmente könnten beispielsweise in einem hexagonalen oder rechteckigen Muster angebracht werden. Auch eine Fertigung des Auskoppelelements als mehrfach belichtetes (gemultiplextes) Volumenhologramm ist denkbar. Im Falle von solchen mehrfach belichteten Volumenhologrammen ist es auch möglich, dass sich einzelne Segmentierungsbereiche räumlich teilweise oder vollständig überlappen.

In solchen Ausführungsbeispielen werden die einzelnen ebenen Wellen vom optischen System des Auges fokussiert und erzeugen auf der Netzhaut ein Punkteraster, welches das aktive Augenimplantat dann mit Energie versorgen kann.

Um zu vermeiden, dass die Strahlenleistungsdichte auf der Retina die vorgegebenen Grenzwerte übersteigt, können zusätzliche Maßnahmen zur Verbreiterung der Fokuspunkte getroffen werden. Einerseits können alle bereits weiter oben diskutierten Ansätze zum Einbringen einer Streufunktion verwendet werden.

Andererseits besteht die Möglichkeit, anstelle von ebenen Wellen eine Vielzahl von Wellenfronten mit einer Restdivergenz auszukoppeln. Die einzelnen Wellenfronten können hierbei sowohl divergent als auch konvergent sein. Hierdurch verschiebt sich der Fokus der Teilwellen vor oder hinter die Retina und die Strahlungsleistungsdichte sinkt. Gleichzeitig kann dies dazu führen, dass die Netzhaut homogener ausgeleuchtet wird.

Im Beispiel der Fig. 4B weist die räumliche Verteilung des zweifach expandierten Lichts zusätzlich einen räumlichen Versatz auf. Im Beispiel der Fig. 4B ist dies exemplarisch mit einem räumlichen Versatz der ebenen Welle 370 gezeigt, die einen Versatz entlang der ersten Richtung aufweist und sich in Form der ebenen Welle 370 wiederholt. Aber auch eine Wiederholung in die erste Richtung 510 oder um eine Kombination von erster Richtung und zweiter Richtung sind möglich. Das Bereitstellen von ebenen Wellen mit gleicher Propagationsrichtung durch ein Multiplexing an unterschiedlichen Positionen des Brillenglases 101 kann den Vorteil haben, dass eine Energieversorgung des aktiven Augenimplantats auch für starke Rollbewegungen des Auges gewährleistet sein kann.

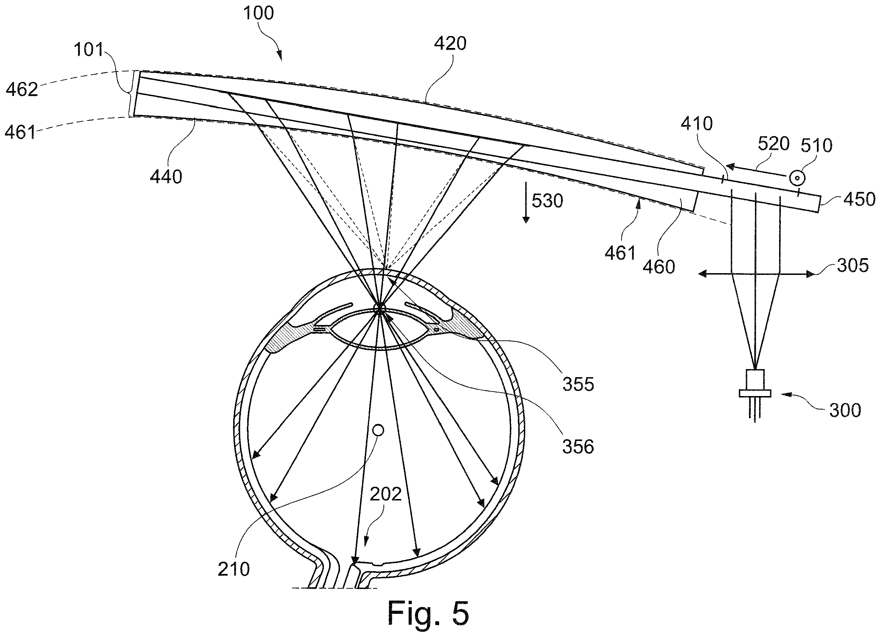

Fig. 5 zeigt ein weiteres Ausführungsbeispiel mit einem von transparentem Material umgebenen Wellenleiter.

Das in Fig. 5 gezeigte Brillenglas 101 umfasst einen Wellenleiter 450 und transparentes Material 460. Die nachfolgend im Zusammenhang mit der Fig. 5 erläuterten Eigenschaften werden im Zusammenhang mit einem modifizierten Ausführungsbeispiel der Fig. 3A erläutert. Aber auch eine Kombination mit anderen Ausführungsbeispielen ist möglich.

Der Strahlexpander 400 ist in dem Wellenleiter 450 angeordnet und das Licht wird von der Lichtquelle 300 in den Wellenleiter 450 eingekoppelt. Im gezeigten Beispiel umgibt das transparente Material 460 den Wellenleiter 450 auf der augenzugewandten und der augenabgewandten Seite. Aber auch andere Ausführungsbeispiele sind möglich.

Im gezeigten Beispiel der Fig. 5 kann es sich beim Wellenleiter 450 um einen planen Wellenleiter aus Glas handeln. Dann kann das transparente Material 460 als Splitterschutz dienen, wie zuvor beschrieben. Im gezeigten Beispiel weisen sowohl augenabgewandte Seite als auch augenzugewandte Seite des transparenten Materials eine Wölbung 461, 462 auf. Hierdurch weist das transparente Material eine Brechkraft auf. Die Wölbungen sind hier nur exemplarisch, auch andere aus der Brillenoptik bekannte Glasformen können verwendet werden, beispielsweise beliebige Kombinationen von konkaven und konvexen Linsenformen und komplexere Formen, beispielsweise Gleitsichtbrillenformen oder Linsenformen die einen Astigmatismus kompensieren können.

Ist eine solches transparentes Material vorhanden, ist dies bei dem optischen Design des Strahlexpanders zu berücksichtigen, da das Licht an den Grenzflächen zwischen Wellenleiter 450 und transparentem Material 460 sowie an der Grenzfläche von transparentem Material 460 und der Luft gebrochen wird. Im gezeigten Beispiel wird die zerstreuende Wirkung der inneren Wölbung 461 durch eine Erhöhung der Krümmung der durch den Wellenleiter generierten Wellenfront kompensiert. Ohne diese Kompensation würde der Fokus nicht, wie in Fig. 3A, im Bereich der Pupille liegen, wie durch die gestrichelten Linien 355 angedeutet. Dadurch, dass diese Brechungen berücksichtigt werden, liegt der Fokuspunkt wieder an der richtigen Stelle 356.

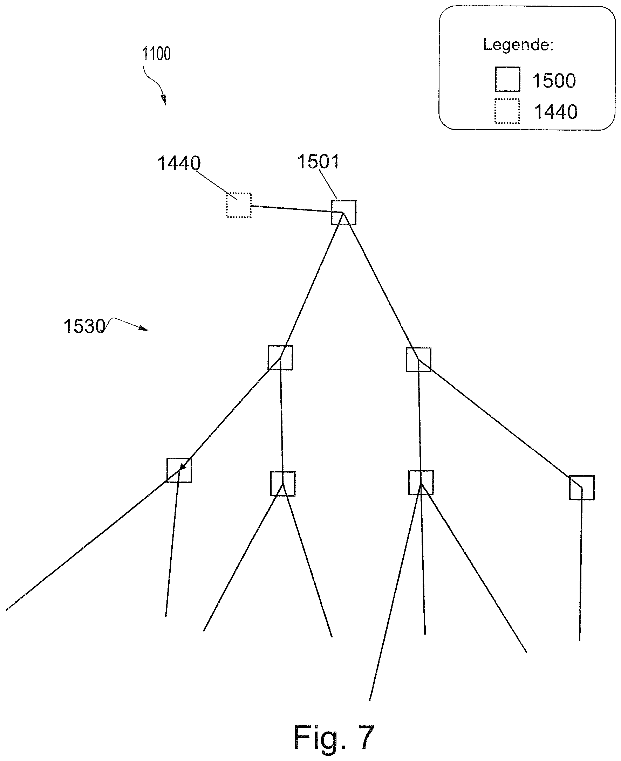

Expansionselemente wie die oben beschriebenen Expansionselemente können auch eine Baumstruktur bilden. Eine derartige Vorrichtung 1100 ist in der Fig. 7 veranschaulicht.

Fig. 7 zeigt hierbei eine Detailansicht einer Vorrichtung 1100, die wie für manche der obigen Ausführungsbeispiele beschrieben in einem als Lichtwellenleiter dienenden Brillenglas angeordnet sein kann. Es wird eine Vielzahl von Expansionselementen 1500 schematisch gezeigt, wobei ein erstes Expansionselement Licht direkt oder indirekt (über andere Elemente) von einer Lichtquelle 1440 empfängt. Die weiteren Elemente der Vielzahl von Expansionselementen 1500 weisen eine Baumstruktur 1530 auf.

Die Expansionselemente 1500 können hierbei sowohl zur Weiterleitung von Licht im Brillenglas an ein nächstes Expansionselement 1500 in der Baumstruktur als auch zur Auskopplung von Licht aus dem Brillenglas, um Licht zu einem Auge zu lenken, eingerichtet sein. Durch die Baumstruktur wird der Freiheitsgrad beim Design einer Leuchtverteilung des Lichts, die von der Vorrichtung 1100 erzeugt werden kann, weiter gesteigert.

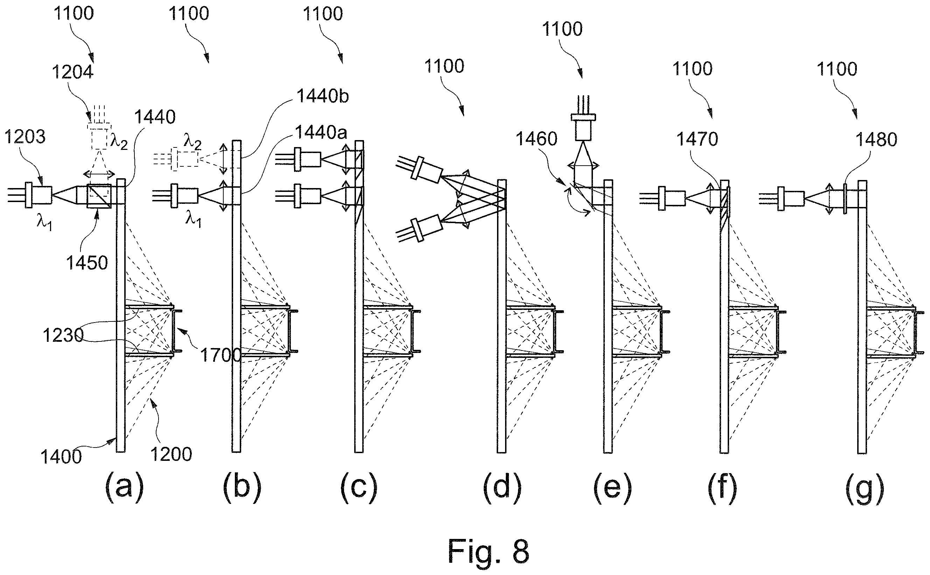

Fig. 8 zeigt Vorrichtungen gemäß verschiedener Ausführungsbeispiele, die mehrkanalig und/oder schaltbar sind. Hier kann zum Beispiel Licht von zwei verschiedenen Lichtquellen verwendet werden. Licht von einer Lichtquelle kann beispielsweise zum Versorgen eines Augenimplantats mit Energie wie oben beschrieben dienen, während Licht von einer anderen Lichtquelle anderen Zwecken dient, beispielsweise zur Beleuchtung oder Projektion von Informationen. Bei anderen Ausführungsbeispielen können verschiedene Wellenlängen zum Versorgen von Augenimplantaten bereitgestellt werden. Bei wieder anderen Ausführungsbeispielen kann Licht mit verschiedenen Leuchtverteilungen zum Versorgen von Augenimplantaten bereitgestellt werden. Beispielsweise kann mittels eines Eyetrackers eine Blickrichtung des Auges erfasst werden und abhängig von der Blickrichtung eine Leuchtverteilung ausgewählt werden, um das Augenimplantat bei der jeweiligen Blickrichtung effizient mit Energie zu versorgen. Beispielsweise kann eine Blickrichtung des Auges mit einem so genannten Eyetracker überwacht werden und die Leuchtverteilung in Abhängigkeit von der Blickrichtung gewählt werden, um eine Energieversorgung des Implantats zu optimieren und/oder sicherzustellen, dass ein möglichst großer Teil der abgestrahlten Energie das Augenimplantat erreicht.

Hierdurch kann auch eine Versorgung des Augenimplantats über einen großen Blickwinkelbereich (field of view) erfolgen.

Hierbei zeigen die Unterfiguren Fig. 8 (a) bis Fig. 8 (g) verschiedene Beispiele mehrkanaliger bzw. schaltbarer Vorrichtungen, die eingerichtet sind, unterschiedliche Leuchtverteilungen bereitzustellen, beispielsweise zum Versorgen eines Augenimplantats mit Energie.

Nachfolgend werden anhand von Vorrichtungen 1100 bei (a) bis (g) verschiedene Konzepte mehrkanaliger Wellenleitersysteme beschrieben. Die Konzepte können sich hohe spektrale und/oder angulare Selektivität von Expansionselementen, die als diffraktives Elemente, beispielsweise mit Volumenhologrammen oder anderen mikrostrukturierten optischen Elementen, implementiert sind, zunutze machen, um mehrere Strahlenbündel unabhängig voneinander innerhalb desselben Volumens eines als ein Lichtleiter 1400 dienenden Brillenglases übertragen zu können. Unter einer hohen spektralen Selektivität wird hierbei der Abfall der Effizienz des Elements beispielsweise um 50 % Halbwertsbreite, manchmal auch als Breite bei halber Höhe (Englisch: Full Width at Half Maximum, FWHM) bei Wellenlängenabweichungen von der Designwellenlänge beispielsweise <40 nm, beispielsweise <10 nm, verstanden.

Unter einer hohen angularen Selektivität wird ein Abfall der Effizienz des Elements um 50 % FWHM bei einer Abweichung des Strahleinfallswinkels von einem Designwinkel, für den das jeweilige optische Element ausgelegt ist, beispielsweise um ein zugehöriges Eingangslichtbündel aus diesem Winkel zu empfangen, von beispielsweise < 10°, beispielsweise < 2° verstanden. In solchen Fällen, aber nicht darauf beschränkt, können mehrere Strahlenbündel mit unterschiedlichen Richtungen und/oder Wellenlängen innerhalb des gleichen Volumens des Lichtwellenleiters 1400 propagieren und können selektiv von zugeordneten, manchmal auch als "passenden" beschriebenen, optischen Elementen gekoppelt werden und weitergeleitet werden. Mit anderen Worten können innerhalb eines identischen Volumens des Lichtleiters 1400 selektiv wirkende Expansionselemente bereitgestellt werden. Expansionselemente können dabei eingerichtet sein, mindestens ein zugehöriges Eingangslichtbündel mit einem Eingangsstrahlprofil zu empfangen und eine Vielzahl von zugehörigen Ausgangslichtbündeln mit jeweiligen Ausgangsstrahlprofilen bereitzustellen, beispielsweise ein Lichtbündel aus einem Brillenglas auszukoppeln und ein anderes Lichtbündel in dem Brillenglas weiterzuleiten. Diese Expansionselemente können in Superposition arbeiten und das Licht für unterschiedliche Charakteristiken, beispielsweise Einfallswinkel, in unterschiedliche Leuchtverteilungen umwandeln. Dies wird manchmal auch als Multiplexing beschrieben, beispielsweise als spektrales Multiplexing, wenn die optischen Elemente, beispielsweise Volumenhologramme, so eingerichtet sind, dass sie verschiedene Kopplungsverhalten für verschiedene spektrale Eigenschaften des Lichts aufweisen. Auch andere Arten von Multiplexing sind möglich, beispielsweise Winkel- oder polarisationsabhängiges Multiplexing, sowie Kombinationen davon.

Nachfolgend wird diese Grundidee am Beispiel von Seitenansichten von Vorrichtung 1100 in Fig. 8 kurz erläutert. Es werden hierbei nur maximal zwei Lichtquellen beispielhaft gezeigt, dies ist natürlich nicht als einschränkend auszulegen, auch komplexere Systeme zum Beispiel mit mehr als zwei Lichtquellen sind möglich.

Die Vorrichtung bei (a) zeigt eine Vorrichtung 1100, die eingerichtet ist, Licht von einer ersten Lichtquelle 1203 mit einer ersten Wellenlänge λ1 und Licht von einer zweiten Wellenlänge λ2 von einer zweiten Lichtquelle 1204 zu empfangen, und für jede empfangene Wellenlänge eine Leuchtverteilung 1200 zu erzeugen. Im gezeigten Beispiel umfasst die Leuchtverteilung 1200 eine Leuchtverteilung, welche sich aus der Leuchtverteilung 1200 z.B. zum Versorgen eines Augenimplantats mit Energie sowie einer Leuchtverteilung von Fixationsmarken 1230 zusammensetzt. Ein solcher Aufbau kann den Vorteil haben, dass es möglich ist, mit demselben Lichtwellenleiter 1400 in verschiedenen Wellenlängenbereichen für verschiedene Zwecke verschiedene Leuchtverteilungen bereitzustellen, im gezeigten Beispiel beispielsweise die Fixationsmarken 230 bei einer Wellenlänge λ2 der zweiten Lichtquelle 1204 im sichtbaren Bereich und Infrarotlicht bei einer Wellenlänge λ1 der ersten Lichtquelle 1203 im Infraroten. Es können auch beide Lichtquellen im Infraroten bei verschiedenen Wellenlängen senden, insbesondere zur Erzeugung verschiedener Leuchtverteilungen zum Versorgen des Augenimplantats mit Energie.

Fig. 8 (b) zeigt eine alternative Implementierung der Vorrichtung der Fig. 8 (a) mit einem anders gestalteten Einkopplungselement 1440. In Bezug auf die Fig. 8 beschriebene Einkopplungselemente können mit diffraktiven Elementen, insbesondere vergrabenen diffraktiven Elementen, realisiert sein. In diesem Ausführungsbeispiel umfasst das Einkopplungselement 1440 zwei verschiedene Bereiche, wobei ein erster Einkopplungsbereich 1440A eingerichtet ist, das Licht der ersten Lichtquelle 1203 einzukoppeln, und ein zweiter Einkopplungsbereich 1440B eingerichtet ist, das Licht der zweiten Lichtquelle 1203 in den Lichtwellenleiter 1400 einzukoppeln.

Die Fig. 8 (c) bis Fig. 8 (g) zeigen verschiedene Möglichkeiten zur Realisierung von schaltbaren Systemen und/oder Systemen, die mehrere Leuchtverteilungen in Überlagerung, manchmal auch als Superposition bezeichnet, ermöglichen.

Im Beispiel der Fig. 8 (c) sind die Lichtquellen 1203, 1204 lateral versetzt angeordnet und werden von Einkopplungselementen 1440A, 1440B an unterschiedlichen Stellen in den Lichtwellenleiter 4100 eingekoppelt.

Die jeweiligen zugehörigen Einkopplungselemente 1440A, 1440B können hierbei so ausgestaltet sein, dass selbst bei gleichartigen Lichtquellen 1203, 1204 unterschiedliche Einkopplungen in den Lichtwellenleiter 1400 erreicht werden, beispielsweise unterschiedliche Einkopplungswinkel. Somit kann die Vorrichtung 1100 eingerichtet sein, zwei Leuchtverteilungen, im gezeigten Beispiel eine jeweilige Leuchtverteilung pro Lichtquelle, bereitzustellen. Diese Leuchtverteilungen können in manchen Beispielen unabhängig voneinander gewählt werden, beispielsweise aufgrund der zuvor beschriebenen Winkelselektivität und/oder Wellenlängenselektivität der verwendeten optischen Elemente.

Fig. 8 (d) zeigt eine Variation der Fig. 8 (c), wobei die beiden Lichtquellen 1204, 1203 unter unterschiedlichen Winkeln auf ein Einkopplungselement 1440 treffen. Dieses ist eingerichtet, die beiden Lichtquellen unabhängig voneinander in den Lichtwellenleiter 1400 einzukoppeln. Im Beispiel der Fig. 8 (e) ist nur eine Lichtquelle 1203 vorhanden. Der Einfallwinkel des Lichtes der Lichtquelle 1203 wird hierbei durch einen Scanspiegel 1460 variiert, wodurch eine schaltbare Leuchtverteilung entsteht. Im Beispiel der Fig. 8 (f) ist ein schaltbares optisches Element 1470, beispielsweise ein schaltbares Hologramm, im Lichtwellenleiter 1400 vorhanden. Auch hierdurch kann eine Superposition verschiedener Leuchtverteilungen erreicht werden. Im Beispiel der Fig. 8 (g) verändert ein polarisationsveränderndes Element 1480 die Polarisationseigenschaften des Lichts von der Lichtquelle1 203. Die optischen Elemente der Vorrichtung 1100 können polarisationsabhängige Eigenschaften aufweisen, sodass auch durch eine Variation der Polarisation des einfallenden Lichtes auf die Vorrichtung 1100 unterschiedliche Leuchtverteilungen herbeigeführt werden können.

Die in den Fig. 8 (a) bis Fig. 8 (g) gezeigten Beispiele können auch miteinander kombiniert werden. So können beispielswiese verschiedene Lichtquellen mit unterschiedlichen Polarisationsrichtungen - entsprechend dem Beispiel der Fig. 8 (g) - mit einem Scanspiegel - wie in Fig. 8 (e) gezeigt - kombiniert werden. Aber auch beliebige andere Kombinationen der gezeigten Elemente und Vorgehensweisen sind möglich.

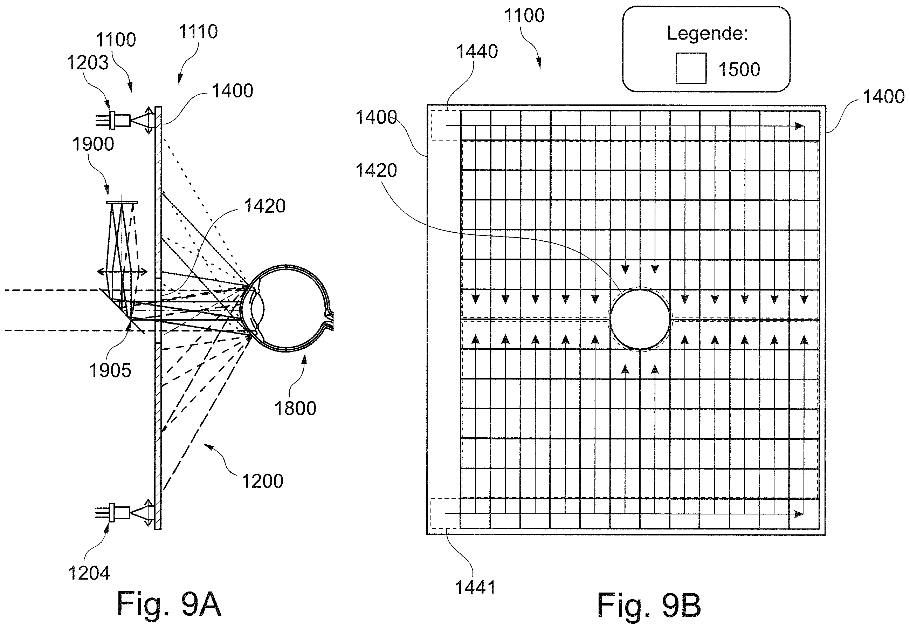

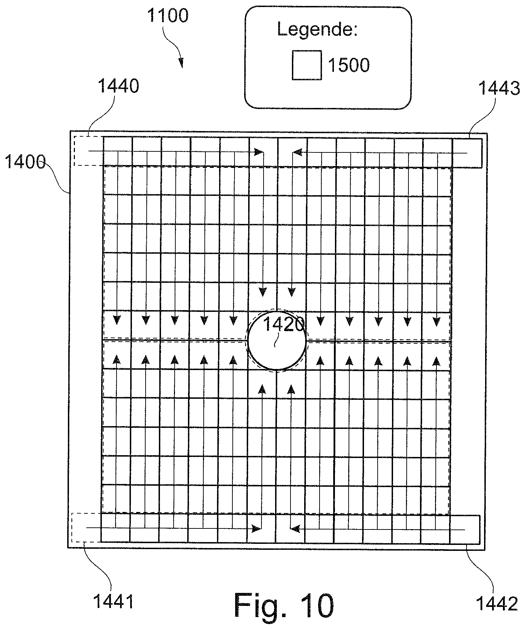

Bei manchen Ausführungsbeispielen kann eine Öffnung in dem Brillenglas wünschenswert sein, beispielsweise um Untersuchungen des Auges durchführen zu können. Um dennoch eine passende Leuchtverteilung von Licht zum Versorgen eines Augenimplantats mit Energie bereitzustellen, kann eine solche Vorrichtung dann wie unter Bezugnahme auf Fig. 9A, 9B und 10 erläutert ausgestaltet sein.

Fig. 9A zeigt eine Seitenansicht einer Vorrichtung 1100 gemäß einem Ausführungsbeispiel. Fig. 9B zeigt eine Frontalansicht der Vorrichtung 1100.

Mit der Vorrichtung 1100 wird eine Leuchtverteilung 1200 zur Versorgung eines Augenimplantats eines Auges 1800 mit Energie bereitgestellt. Mit einer schaltbaren Vorrichtung wie unter Bezugnahme auf Fig. 8 erläutert kann bei manchen Ausführungsbeispielen schaltbar auf eine Leuchtverteilung zum Beleuchten des Auges für eine Untersuchung, beispielsweise für eine keratometrischen Vermessung der Hornhaut des Auges 800 bereitgestellt. Das Auge kann zudem durch eine Aussparung 1420 in einem als Lichtwellenleiter 1400 dienenden Brillenglas untersucht werden. Beispielsweise kann im Falle der oben erwähnten keratometrischen Untersuchung das von der Hornhaut des Auges 1800 reflektierte Licht wird von einer Detektionsvorrichtung 1900 entlang eines Nachweisstrahlengangs 1905 nachgewiesen und kann anschließend analysiert werden, um auf die Topologie der Hornhaut zu schließen. Der Lichtwellenleiter 1400 der Vorrichtung 1100 weist wie erwähnt die Aussparung 1420 auf. Um trotz der Aussparung 1420 eine zur Versorgung eines Augenimplantats mit Energie oder ggfs. auch zur Beleuchtung wie zur Keratometrie geeignete Leuchtverteilung1200 zu erreichen, die möglichst ein gesamtes Blickfeld des Auges 800 abdeckt oder das gesamte zu untersuchende Auge beleuchtet, wird das Licht von zwei Lichtquellen 1203, 1204 bereitgestellt und von zwei Einkopplungselementen 1440, 1441 eingekoppelt. Ausgehend von den jeweiligen Einkopplungselementen 1440, 1441, wird das Licht über eine Vielzahl von Expansionselementen wie oben beschrieben repliziert und in Richtung des Auges 1800 als Leuchverteilung 1200 ausgekoppelt.

In dem gezeigten Beispiel der Vorrichtung ist die Flächennormale des Lichtwellenleiters 1400 parallel zu einer Hauptsehachse des Auges 8100 angeordnet. In anderen Ausführungsbeispielen kann die Normale des Lichtwellenleiters aber auch gerade nicht parallel zu der Hauptsehachse des Auges 1800 angeordnet sein. Hierdurch können beispielsweise Reflexe reduziert oder vermieden werden.

Fig. 10 zeigt ein weiteres Ausführungsbeispiel einer Vorrichtung 1100 also Abwandlung der Vorrichtung der Fig. 9A, 9B.

In der Vorrichtung 1100 der Fig. 10 sind vier Einkopplungselemente 1440 bis 1443 vorhanden. Die Vielzahl von Expansionselementen 1500 sind hierbei so miteinander gekoppelt, dass eine Leuchtverteilung wie die Leuchtverteilung 1200 der Fig. 7A durch die Vielzahl von Expansionselementen 1500 bereitgestellt werden kann.

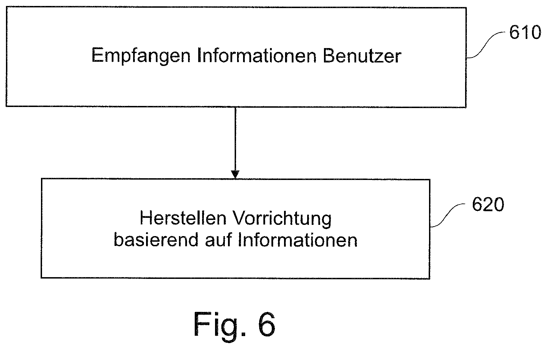

Es kann vorteilhaft sein, die zuvor beschriebenen Vorrichtungen 100, 1100 individuell für einzelne Benutzer oder Gruppen von Benutzern anzupassen. Solche Verfahren werden nachfolgend im Zusammenhang mit Fig. 6 beschrieben.

Fig. 6 zeigt ein Ablaufdiagramm für ein Verfahren gemäß verschiedener Ausführungsbeispiele.

Hierbei wird ein Verfahren zum Herstellen einer nutzerspezifischen Brille beschrieben.

Bei 610 erfolgt ein Empfangen von Kopfgeometrieinformationen des Benutzers.

Die Kopfgeometrie, insbesondere Kopfbreite und Pupillendistanz, variieren stark von Nutzer zu Nutzer. Deshalb kann es vorteilhaft sein, unterschiedlichen Nutzern unterschiedlich breite Brillengestelle bereitzustellen und das Brillenglas nutzerspezifisch zu beranden. Beispielsweise können die Brillengestelle genutzt werden, um darin die in den Figuren 3A, 3B, 4A, 4B und 5 gezeigten Lichtquellen 300 anzuordnen. Um für verschiedene Kopfgeometrien jeweils ästhetisch aussehende Brillengestelle zu realisieren, kann es vorteilhaft sein, geometrische Parameter und optische Eigenschaften der Vorrichtung zu variieren. Beispielsweise kann der horizontale Abstand der im Bügel untergebrachten Lichtquelle zum Auge variiert werden.

Bei 620 erfolgt ein Herstellen einer Vorrichtung wie zuvor beschrieben basierend auf den Kopfgeometrieinformationen.

Die zuvor beschriebenen Vorrichtungen können hierbei so ausgestaltet sein, dass eine solche Anpassung, wie bei 620 beschrieben, besonders einfach zu erreichen ist:
In den Beispielen, bei denen die Einkopplungs-, Umlenk- und/oder Expansionselemente mittels Volumenhologrammen realisiert werden, kann es möglich sein, eine Anpassung für einen einzelnen Benutzer vorzunehmen, indem ein nutzerspezifischen Lateralversatz des Belichtungsstrahls bei der Herstellung der Vorrichtung verwendet wird, was dazu führt, dass das Einkopplungselement an eine für den Nutzer geeignete Stelle geschrieben wird.

Wie bereits erläutert, dienen die obigen Ausführungsbeispiele lediglich der Veranschaulichung und sind nicht als einschränkend auszulegen.

## Patentansprüche

1. Vorrichtung (100, A, B, 1100) mit einem Brillenglas (101) zur Versorgung eines aktiven Augenimplantats (202) in einem Auge (201; 1800) eines Benutzers (200) mit Energie, umfassend:
eine Lichtquelle (300),
einen Strahlexpander (400), der in oder auf dem Brillenglas (101) angeordnet ist,
wobei der Strahlexpander (400) ein erstes Expansionselement (410) und ein zweites Expansionselement (420) umfasst,
wobei die Vorrichtung eingerichtet ist, Licht (310) von der Lichtquelle (300) in das Brillenglas einzukoppeln und zu dem ersten Expansionselement zu leiten,
wobei das erste Expansionselement eingerichtet ist, das Licht zu empfangen, entlang einer ersten Richtung (510) aufzuweiten und zumindest einen Teil des Lichts als expandiertes Licht (320) in einer zweiten Richtung (520) an das zweite Expansionselement (420) zu leiten, wobei die zweite Richtung von der ersten Richtung verschieden ist und
wobei das zweite Expansionselement eingerichtet ist, das expandierte Licht (320) zu empfangen, entlang der zweiten Richtung aufzuweiten und über eine Abgabefläche (440) als zweifach expandiertes Licht (330) in eine dritte Richtung (530) bereitzustellen, wobei die dritte Richtung (530) zumindest teilweise nicht in dem Brillenglas verläuft,
wobei das zweite Expansionselement eingerichtet ist, das zweifach expandierte Licht (330) effektiv zu fokussieren.

2. Vorrichtung (100, A, B, 1100) nach Anspruch 1, wobei das effektive Fokussieren umfasst, dass das zweifach expandierte Licht (330) auf eine gedachte Fokusfläche (340) zwischen Abgabefläche (440) und dem aktiven Augenimplantat (202) entlang der dritten Richtung (530) gelenkt wird, wobei die gedachte Fokusfläche (340) kleiner als die Abgabefläche (440) ist.

3. Vorrichtung nach einem der vorherigen Ansprüche, wobei der Strahlexpander (400) ein drittes Expansionselement (430) umfasst und wobei das erste Expansionselement ferner eingerichtet ist, einen weiteren Teil des expandierten Lichts (322) zusätzlich in eine vierte Richtung (540) an das dritte Expansionselement (430) zu leiten, wobei die vierte Richtung von der ersten, zweiten und dritten Richtung verschieden ist, und
wobei das dritte Expansionselement (430) eingerichtet ist, den weiteren Teil des expandierten Lichts (320) zu empfangen, entlang der vierten Richtung (540) aufzuweiten und über die Abgabefläche (440) als einen weiteren Teil des zweifach expandierten Lichts in eine fünfte Richtung (550) bereitzustellen, wobei die fünfte Richtung (550) zumindest teilweise nicht in dem Brillenglas verläuft, und
wobei das dritte Expansionselement (430) eingerichtet ist, den weiteren Teil des zweifach expandierten Lichts (332) effektiv zu fokussieren.

4. Vorrichtung (100, A, B, 1100) nach einem der vorherigen Ansprüche, wobei das zweifach expandierte Licht (330) ein konvergentes Lichtbündel (350) mit einem Fokuspunkt (360) umfasst.

5. Vorrichtung (100, A, B, 1100) nach einem der vorherigen Ansprüche, wobei die Vorrichtung (100) eine Steuerung umfasst, wobei die Steuerung eingerichtet ist, eine Blickrichtung des Benutzers zu bestimmen, und
wobei die Vorrichtung (100) eingerichtet ist, die effektive Fokussierung des zweifach expandierten Lichts in Reaktion auf eine Änderung der Blickrichtung des Auges anzupassen.

6. Vorrichtung (100, A, B, 1100) nach Anspruch 5, wobei die Vorrichtung (100) eine Aufnahmevorrichtung und einen Scanspiegel umfasst, wobei die Steuerung eingerichtet ist, basierend auf einer Information der Aufnahmevorrichtung die Blickrichtung des Benutzers zu bestimmen und basierend auf der Blickrichtung den Scanspiegel anzusteuern.

7. Vorrichtung (100, A, B, 1100) nach Anspruch 5 oder 6, wobei die Vorrichtung (100, A, B, 1100) eingerichtet ist, das Anpassen der effektiven Fokussierung des zweifach expandierten Lichts mittels einer Verdrehung und/oder einer lateralen Verschiebung von einem Element herbeizuführen, wobei das Element aus einer Gruppe ausgewählt ist, welche umfasst:
die Lichtquelle (300),
eine Kollimationsoptik (305).

8. Vorrichtung (100, A, B, 1100) nach einem der vorherigen Ansprüche, wobei die Vorrichtung (100) eingerichtet ist, die effektive Fokussierung in eine sechste Richtung (560) zu vergrößern, wobei die sechste Richtung senkrecht zur dritten Richtung (530) ist.

9. Vorrichtung (100, A, B, 1100) nach einem der vorherigen Ansprüche, wobei das zweifach expandierte Licht (330) mehrere räumlich begrenzte Wellen (370, 380, 390) umfasst, die jeweils von verschiedenen Bereichen der Abgabefläche zumindest teilweise in Richtung der dritten Richtung propagieren, wobei sich die mehreren räumlich begrenzten ebenen Wellen zumindest teilweise kreuzen und/oder divergieren.

10. Vorrichtung (100, A, B, 1100) nach einem der vorherigen Ansprüche, wobei die Lichtquelle eine Laserdiode (300) mit einem Astigmatismus umfasst und das Brillenglas eingerichtet ist, eine anisotrope Divergenzanpassung des Lichts von der Laserdiode vorzunehmen.

11. Vorrichtung (100, A, B, 1100) nach einem der vorherigen Ansprüche, wobei das Brillenglas einen Wellenleiter (450) und transparentes Material (460) umfasst,
wobei der Strahlexpander (400) in oder auf dem Wellenleiter angeordnet ist und das Licht von der Lichtquelle in den Wellenleiter (450) eingekoppelt wird,
wobei das transparente Material den Wellenleiter auf mindestens einer Seite zumindest teilweise umgibt.

12. Vorrichtung (100, A, B, 1100) nach Anspruch 11, wobei das transparente Material eine Wölbung (461) auf einer dem Auge des Benutzers zugewandten Seite des zweiten Expansionselement aufweist, wobei das transparente Material eingerichtet ist, die effektive Fokussierung des Lichts durch Brechung zu modifizieren.

13. Vorrichtung (1100) nach einem der vorherigen Ansprüche, wobei die Lichtquelle mindestens eines der folgenden Elemente umfasst:
zwei Einzellichtquellen (1203, 1204), die eingerichtet, sind Licht in unterschiedlichen Richtungen und/oder in unterschiedlichen Wellenlängenbereichen und/oder an unterschiedliche Beleuchtungspositionen mindestens einen Einkopplungselements (1440; 1441-1443) zum Einkoppeln des Lichts in das Brillenglas (101) bereitzustellen,
einen Strahlteiler (1450),
einen Scanspiegel (1460),
ein schaltbares Element (1470),
wobei die Vorrichtung eingerichtet ist, in Abhängigkeit von einer Blickrichtung des Auges (1800) zwischen mindestens zwei verschiedenen Leuchtverteilungen von Licht, das aus dem Brillenglas (101) austritt, umzuschalten.

14. Verfahren zum Herstellen einer nutzerspezifischen Brille, wobei das Verfahren umfasst:
Empfangen von Kopfgeometrieinformationen des Benutzers,
Herstellen einer Vorrichtung gemäß einem der vorherigen Ansprüche basierend auf den Kopfgeometrieinformationen.

15. Verfahren nach Anspruch 14, wobei die Vorrichtung eine Vorrichtung nach Anspruch 13 umfasst, wobei das Verfahren umfasst:
Aktivieren eines Teils des Brillenglases, um das Einkopplungselement (405) bereitzustellen.

16. Verfahren nach einem der Ansprüche 14 oder 15, wobei das Einkopplungselement ein Oberflächengitter und/oder ein Volumenhologramm umfasst, wobei das Einkopplungselement für einen Parameterbereich von Kopfgeometrieinformationen eingerichtet ist, und eine Einkopplungsfläche aufweist, die größer ist als eine Lichtquelleneinkopplungsfläche der Lichtquelle auf dem Einkopplungselement,
und wobei das Verfahren ein Positionieren der Lichtquelle basierend auf den Kopfgeometrieinformationen umfasst, wodurch die Lichtquelleneinkopplungsfläche in Bezug zur Einkopplungsfläche fest angeordnet wird.

17. Verfahren nach Anspruch 16, wobei das Verfahren weiter umfasst:
Deaktivieren eines Teils der Einkopplungsfläche, wobei das Deaktivieren mit einer oder
mehreren der folgenden Schritte erfolgt:
- elektrisches Beeinflussen eines elektrischen Gitters in der Einkopplungsfläche,
- Abtragen eines UV-Lacks in einem Bereich der Einkopplungsfläche,
- lokales Einbringen eines Materials in ein Oberflächengitter, wobei das Material eine ähnliche Brechzahl wie ein Material des Einkopplungselements aufweist,
- Zerstören einer Wirkung eines Teils eines Hologramms mittels Strahlung, insbesondere elektromagnetischer Strahlung und/oder Temperatur.

## Claims

1. Apparatus (100, A, B, 1100) with a spectacle lens (101) for supplying power to an active ocular implant (202) in an eye (201; 1800) of a user (200), comprising:
a light source (300),
a beam expander (400) arranged in or on the spectacle lens (101),
wherein the beam expander (400) comprises a first expansion element (410) and a second expansion element (420),
wherein the apparatus is set up to input couple light (310) from the light source (300) into the spectacle lens and guide said light to the first expansion element,
wherein the first expansion element is set up to receive the light, expand said light along a first direction (510) and guide at least some of the light as expanded light (320) in a second direction (520) to the second expansion element (420), wherein the second direction differs from the first direction, and
wherein the second expansion element is set up to receive the expanded light (320), expand said expanded light along the second direction and provide said expanded light as twice expanded light (330) in a third direction (530) via an emission surface (440), wherein the third direction (530) at least partly does not extend in the spectacle lens,
wherein the second expansion element is set up to effectively focus the twice expanded light (330).

2. Apparatus (100, A, B, 1100) according to Claim 1, wherein effective focusing comprises steering the twice expanded light (330) to an imaginary focal surface (340) between the emission surface (440) and the active ocular implant (202) along the third direction (530), wherein the imaginary focal surface (340) is smaller than the emission surface (440).

3. Apparatus according to any one of the preceding claims, wherein the beam expander (400) comprises a third expansion element (430) and wherein the first expansion element is further set up to additionally guide a further portion of the expanded light (322) to the third expansion element (430) in a fourth direction (540), wherein the fourth direction differs from the first, second and third direction, and
wherein the third expansion element (430) is set up to receive the further portion of the expanded light (320), expand said expanded light along the fourth direction (540) and provide said expanded light as a further portion of the twice expanded light in a fifth direction (550) via the emission surface (440), wherein the fifth direction (550) at least partly does not extend in the spectacle lens, and
wherein the third expansion element (430) is set up to effectively focus the further portion of the twice expanded light (332).

4. Apparatus (100, A, B, 1100) according to any one of the preceding claims, wherein the twice expanded light (330) comprises a convergent light beam (350) with a focal point (360).

5. Apparatus (100, A, B, 1100) according to any one of the preceding claims, wherein the apparatus (100) comprises a controller, wherein the controller is set up to determine a line of sight of the user, and
wherein the apparatus (100) is set up to adapt the effective focusing of the twice expanded light in response to a change in the line of sight of the eye.

6. Apparatus (100, A, B, 1100) according to Claim 5, wherein the apparatus (100) comprises a recording apparatus and a scanning mirror, wherein the controller is set up to determine the line of sight of the user on the basis of information from the recording apparatus and to control the scanning mirror on the basis of the line of sight.

7. Apparatus (100, A, B, 1100) according to Claim 5 or 6, wherein the apparatus (100, A, B, 1100) is set up to bring about the adaptation of the effective focusing of the twice expanded light by means of a twist and/or a lateral displacement of an element, wherein the element is selected from a group comprising:
the light source (300),
a collimation optical unit (305).

8. Apparatus (100, A, B, 1100) according to any one of the preceding claims, wherein the apparatus (100) is set up to increase the effective focusing in a sixth direction (560), wherein the sixth direction is perpendicular to the third direction (530).

9. Apparatus (100, A, B, 1100) according to any one of the preceding claims, wherein the twice expanded light (330) comprises a plurality of spatially restricted waves (370, 380, 390) which each propagate at least in part from different regions of the emission surface in the direction of the third direction, wherein the plurality of spatially restricted plane waves at least partly cross and/or diverge.

10. Apparatus (100, A, B, 1100) according to any one of the preceding claims, wherein the light source comprises a laser diode (300) with an astigmatism and the spectacle lens is set up to undertake an anisotropic divergence adjustment of the light from the laser diode.

11. Apparatus (100, A, B, 1100) according to any one of the preceding claims, wherein the spectacle lens comprises a waveguide (450) and transparent material (460),
wherein the beam expander (400) is arranged in or on the waveguide and the light from the light source is input coupled into the waveguide (450),
wherein the transparent material at least partly surrounds the waveguide on at least one side.

12. Apparatus (100, A, B, 1100) according to Claim 11, wherein the transparent material has a curved part (461) on a side of the second expansion element that faces the eye of the user, wherein the transparent material is set up to modify the effective focusing of the light by refraction.

13. Apparatus (1100) according to any one of the preceding claims, wherein the light source comprises at least one of the following elements:
two individual light sources (1203, 1204) which are set up to provide light in different directions and/or in different wavelength ranges and/or at different illumination positions of at least one input coupling element (1440; 1441-1443) for input coupling the light into the spectacle lens (101),
a beam splitter (1450),
a scanning mirror (1460),
a switchable element (1470),
wherein the apparatus is set up to switch between at least two different light distributions of light emerging from the spectacle lens (101) depending on a line of sight of the eye (1800).

14. Method for producing a user-specific pair of spectacles, wherein the method comprises:
receiving head geometry information relating to the user,
producing an apparatus according to any one of the preceding claims on the basis of the head geometry information.

15. Method according to Claim 14, wherein the apparatus comprises an apparatus according to Claim 13, wherein the method comprises:
activating a part of the spectacle lens in order to provide the input coupling element (405).

16. Method according to any one of Claims 14 or 15, wherein the input coupling element comprises a surface grating and/or a volume hologram, wherein the input coupling element is set up for a parameter range of head geometry information and has an input coupling surface that is larger than a light source input coupling surface of the light source on the input coupling element,
and wherein the method comprises a positioning of the light source on the basis of the head geometry information, as a result of which the light source input coupling surface is fixedly arranged in relation to the input coupling surface.

17. Method according to Claim 16, wherein the method further comprises:
deactivating a portion of the input coupling surface, wherein the deactivation is implemented by one or more of the following steps:
- electrically influencing an electrical grating in the input coupling surface,
- ablating a UV resist in a region of the input coupling surface,
- locally introducing a material into a surface grating, wherein the material has a similar refractive index to the material of the input coupling element,
- destroying an effect of some of a hologram by means of radiation, in particular electromagnetic radiation and/or temperature.

## Revendications

1. Dispositif (100, A, B, 1100) comprenant un verre de lunettes (101) et destiné à alimenter en énergie un implant oculaire actif (202) situé dans un œil (201 ; 1800) d'un utilisateur (200), ledit dispositif comprenant :
une source de lumière (300),
un expanseur de faisceau (400) disposé dans ou sur le verre de lunettes (101), l'expanseur de faisceau (400) comprenant un premier élément d'expansion (410) et un deuxième élément d'expansion (420),
le dispositif étant conçu pour injecter par couplage la lumière (310) de la source de lumière (300) dans le verre de lunettes et pour la diriger vers le premier élément d'expansion,
le premier élément d'expansion étant conçu pour recevoir la lumière, l'étaler suivant une première direction (510) et guider au moins une partie de la lumière en tant que lumière expansée (320) dans une deuxième direction (520) vers le deuxième élément d'expansion (420), la deuxième direction étant différente de la première direction et
le deuxième élément d'expansion étant conçu pour recevoir la lumière expansée (320), l'étaler suivant la deuxième direction et la fournir par le biais d'une surface de sortie (440) en tant que lumière doublement expansée (330) dans une troisième direction (530), la troisième direction (530) ne s'étendant pas au moins partiellement dans le verre de lunettes,
le deuxième élément d'expansion étant conçu pour focaliser de manière effective la lumière doublement expansée (330).

2. Dispositif (100, A, B, 1100) selon la revendication 1, la focalisation effective comprenant le fait que la lumière doublement expansée (330) est dirigée sur une surface focale imaginaire (340) située entre la surface de sortie (440) et l'implant oculaire actif (202) suivant la troisième direction (530), la surface focale imaginaire (340) étant plus petite que la surface de sortie (440).

3. Dispositif selon l'une des revendications précédentes, l'expanseur de faisceau (400) comprenant un troisième élément d'expansion (430) et le premier élément d'expansion étant en outre conçu pour diriger une autre partie de la lumière expansée (322) en plus dans une quatrième direction (540) sur le troisième élément d'expansion (430), la quatrième direction étant différente des première, deuxième et troisième directions, et le troisième élément d'expansion (430) étant conçu pour recevoir l'autre partie de la lumière expansée (320), l'étaler suivant la quatrième direction (540) et la fournir par le biais de la surface de sortie (440) en tant que partie supplémentaire de la lumière doublement expansée suivant une cinquième direction (550), la cinquième direction (550) ne s'étendant pas au moins partiellement dans le verre de lunettes, et
le troisième élément d'expansion (430) étant conçu pour focaliser de manière effective l'autre partie de la lumière doublement expansée (332).

4. Dispositif (100, A, B, 1100) selon l'une des revendications précédentes, la lumière doublement expansée (330) comprenant un faisceau lumineux convergent (350) ayant un point focal (360).

5. Dispositif (100, A, B, 1100) selon l'une des revendications précédentes, le dispositif (100) comprenant une commande, la commande étant conçue pour déterminer une direction d'observation de l'utilisateur, et
le dispositif (100) étant conçu pour adapter la focalisation effective de la lumière doublement expansée en réponse à un changement de la direction d'observation de l'œil.

6. Dispositif (100, A, B, 1100) selon la revendication 5, le dispositif (100) comprenant un dispositif de réception et un miroir de balayage, la commande étant conçue pour déterminer la direction d'observation de l'utilisateur sur la base d'une information du dispositif de réception et pour commander le miroir de balayage sur la base de la direction d'observation.

7. Dispositif (100, A, B, 1100) selon la revendication 5 ou 6, le dispositif (100, A, B, 1100) étant conçu pour mettre en œuvre l'adaptation de la focalisation effective de la lumière doublement expansée par une rotation et/ou un coulissement latéral d'un élément, l'élément étant choisi dans un groupe comprenant :
la source de lumière (300),
une optique de collimation (305).

8. Dispositif (100, A, B, 1100) selon l'une des revendications précédentes, le dispositif (100) étant conçu pour augmenter la focalisation effective dans une sixième direction (560), la sixième direction étant perpendiculaire à la troisième direction (530).

9. Dispositif (100, A, B, 1100) selon l'une des revendications précédentes, la lumière doublement expansée (330) comprenant une pluralité d'ondes spatialement limitées (370, 380, 390) qui se propagent chacune depuis différentes zones de la surface de sortie au moins partiellement suivant la troisième direction, la pluralité d'ondes planes spatialement délimitées se croisant et/ou divergeant au moins partiellement.

10. Dispositif (100, A, B, 1100) selon l'une des revendications précédentes, la source de lumière comprenant une diode laser (300) pourvue d'un astigmatisme et le verre de lunettes étant conçu pour effectuer une adaptation de divergence anisotrope de la lumière provenant de la diode laser.

11. Dispositif (100, A, B, 1100) selon l'une des revendications précédentes, le verre de lunettes comprenant un guide d'ondes (450) et un matériau transparent (460),
l'expanseur de faisceau (400) étant disposé dans ou sur le guide d'ondes et la lumière provenant de la source de lumière étant injectée par couplage dans le guide d'ondes (450),
le matériau transparent entourant au moins partiellement le guide d'ondes sur au moins un côté.

12. Dispositif (100, A, B, 1100) selon la revendication 11, le matériau transparent présentant une incurvation (461) sur un côté du deuxième élément d'expansion qui est dirigé vers l'œil de l'utilisateur, le matériau transparent étant conçu pour modifier la focalisation effective de la lumière par réfraction.

13. Dispositif (1100) selon l'une des revendications précédentes, la source de lumière comprenant l'un au moins des éléments suivants :
deux sources de lumière individuelles (1203, 1204) qui sont conçues pour fournir de la lumière dans différentes directions et/ou dans différentes plages de longueurs d'onde et/ou à différentes positions d'éclairage d'au moins un élément d'injection par couplage (1440 ; 1441-1443) destiné à injecter par couplage la lumière dans le verre de lunettes (101),
un séparateur de faisceau (1450),
un miroir de balayage (1460),
un élément commutable (1470),
le dispositif étant conçu pour commuter entre au moins deux distributions lumineuses différentes de la lumière sortant du verre de lunettes (101) en fonction d'une direction d'observation de l'œil (1800).

14. Procédé de fabrication d'un verre de lunettes spécifique à l'utilisateur, le procédé comprenant :
la réception d'informations sur la géométrie de la tête de l'utilisateur,
la fabrication d'un dispositif selon l'une des revendications précédentes sur la base des informations sur la géométrie de la tête.

15. Procédé selon la revendication 14, le dispositif comprenant un dispositif selon la revendication 13, le procédé comprenant :
l'activation d'une partie du verre de lunettes afin de fournir l'élément d'injection par couplage (405).

16. Procédé selon l'une des revendications 14 ou 15, l'élément d'injection par couplage comprenant un réseau de surface et/ou un hologramme volumique, l'élément d'injection par couplage étant conçu pour une plage de paramètres d'informations sur la géométrie de la tête, et une surface d'injection par couplage qui est plus grande qu'une surface d'injection par couplage de la source de lumière sur l'élément d'injection par couplage, et le procédé comprenant le positionnement de la source de lumière sur la base des informations sur la géométrie de la tête de sorte que la surface d'injection par couplage de la source de lumière est fixe par rapport à la surface d'injection par couplage.

17. Procédé selon la revendication 16, le procédé comprenant en outre : la désactivation d'une partie de la surface d'injection par couplage, la désactivation étant effectuée suivant une ou plusieurs des étapes suivantes :
- influer électriquement sur un réseau électrique situé dans la surface d'injection par couplage,
- retirer une peinture UV dans une zone de la surface d'injection par couplage,
- introduire localement un matériau dans un réseau de surface, le matériau ayant un indice de réfraction similaire à celui d'un matériau de l'élément d'injection par couplage,
- détruire un effet d'une partie d'un hologramme au moyen d'un rayonnement, notamment d'un rayonnement électromagnétique et/ou de la température.
